(19) Europäisches Patentamt
European Patent Office
Office européen des brevets

(11) EP 4 253 396 A1

(12) **EUROPEAN PATENT APPLICATION**
published in accordance with Art. 153(4) EPC

(43) Date of publication:
**04.10.2023 Bulletin 2023/40**

(21) Application number: **21898077.9**

(22) Date of filing: **26.11.2021**

(51) International Patent Classification (IPC):
***C07H 19/048*** *(2006.01)* ***A23L 33/13*** *(2016.01)*
***A61K 31/706*** *(2006.01)* ***A61P 3/00*** *(2006.01)*
***A61P 5/00*** *(2006.01)* ***A61P 9/10*** *(2006.01)*
***A61P 43/00*** *(2006.01)*

(52) Cooperative Patent Classification (CPC):
**A23L 33/13; A61K 31/706; A61P 3/00; A61P 5/00; A61P 9/10; A61P 43/00; C07H 19/048**

(86) International application number:
**PCT/JP2021/043333**

(87) International publication number:
**WO 2022/114105 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 JP 2020197381**

(71) Applicant: **Mirailab Bioscience Inc.**
**Tokyo 104-0061 (JP)**

(72) Inventors:
- **TANAKA Tsunemaru**
  **Tokyo 104-0061 (JP)**
- **TANAKA Megumi**
  **Tokyo 104-0061 (JP)**
- **HIRAMATSU Ryuji**
  **Tokyo 104-0061 (JP)**
- **SHIMAMURA Daizo**
  **Tokyo 104-0061 (JP)**

(74) Representative: **Mewburn Ellis LLP**
**Aurora Building**
**Counterslip**
**Bristol BS1 6BX (GB)**

(54) **HIGH-PURITY BETA-NICOTINAMIDE MONONUCLEOTIDE (NMN) AND METHOD FOR PRODUCING SAME**

(57) The present invention addresses the problem of a providing NMN having high purity and a low impurity content. The present invention is a high-purity β-nicotinamide mononucleotide (NMN) which has a purity of 99.0 mass% or higher and in which the amounts of of C11H15NO9P, C10H16N5O10P2, C9H15N3O8P, C9H15N3O7P, C9H16N4O8P, C10H15N5O7P, and C21H27N6O15P2 contained as impurities are equal to or less than a detection limit.

EP 4 253 396 A1

## Description

TECHNICAL FIELD

The present invention relates to high-purity β-nicotinamide mononucleotide (NMN) and a method for producing the same.

BACKGROUND ART

In recent years, research has been actively made on the field of anti-aging medicine (anti-aging field) which regards aging as a disease and attempts to delay the onset itself of aging by treatment. The anti-aging medicine covers a wide range of regions such as endocrine secretion, metabolism, arteriosclerosis, nutrition, locomotor apparatuses, sensory organs, and the like, and is aimed at extending a time to be healthily spent by people through younger physical functions than their current ages, not only targeting aged people but targeting all ages.

Nicotinamide mononucleotide (NMN) is an intermediate for the synthesis of nicotinamide adenine dinucleotide (NAD+). In recent years, NMN has been found to control the activity of a longevity gene "sirtuin" through its conversion to NAD+ and to exhibit anti-aging action when added to mice, and has thus received attention as a substance that suppresses aging (see Non Patent Documents 1 to 3). Although NMN is a substance that is originally produced naturally in the body, the ability to produce NMN in the body is known to be reduced with aging. Hence, anti-aging functional foods, medicaments, and the like containing such NMN have been developed.

Since the anti-aging functional foods, medicaments, and the like containing NMN are taken over a long period, their excellent anti-aging effects as well as high safety is important. Particularly, much remains uncertain about how adversely a product supplemented with NMN rich in impurities affects the body when taken. It is therefore considered desirable that NMN has a purity as high as possible and minimum contents of impurities.

For example, a method of producing NMN by isolating cells (of a yeast, a bacterium, or the like) caused to overexpress nicotinamide phosphoribosyltransferase (Nampt), and culturing the cells in the presence of nicotinamide (NAM) (see Patent Document 1), and a method of producing NMN by using nicotinamide (NAM), ATP, and ribose as starting materials, and reacting these starting materials under the action of catalysts nicotinamide phosphoribosyltransferase (Nampt), ribose phosphate pyrophosphokinase, and ribose kinase (see Patent Document 2) are known as methods for producing NMN.

PRIOR ART DOCUMENTS

Patent Document

Patent Document 1: International Publication No. WO2015/069860
Patent Document 2: International Publication No. WO2017/185549

Non Patent Document

Non Patent Document 1: Yoshino, J., Mills, K.F., Yoon, M.J., & Imai, S. (2011) Cell Metab., 14, 528-536
Non Patent Document 2: Gomes, A.P. et al., (2013) Cell, 155, 1624-1638
Non Patent Document 3: Stein, L.R. & Imai, S. (2014) EMBO J., 33, 1321-1340

SUMMARY OF INVENTION

Technical Problem

The present invention has been made in light of these circumstances. An object of the present invention is to provide NMN having a high purity and low contents of impurities.

Solution to Problem

The present inventors have conducted diligent studies to attain the object and successfully produced high-purity β-nicotinamide mononucleotide (NMN) having a purity of 99.0% by mass or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit, by adding the step of using a dynamic axial compression column (DAC column) or the like to a method for producing NMN. Specifically, the present invention is summarized as follows.

[1] High-purity β-nicotinamide mononucleotide (NMN) having a purity of 99.0% by mass or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit.
[2] A pharmaceutical composition comprising high-purity β-nicotinamide mononucleotide (NMN) according to [1] as an active ingredient.
[3] A composition for aging prevention comprising high-purity β-nicotinamide mononucleotide (NMN) according to [1] as an active ingredient.
[4] A method for producing high-purity β-nicotinamide mononucleotide (NMN), comprising the steps of:

(I) reacting AMP nucleosidase, ribose-phosphate diphosphokinase, adenosine monophosphate (AMP), adenosine triphosphate (ATP), and ribose-5-phosphate (R5P);
(II) reacting nicotinamide phosphoribosyltransferase (Nampt), a reaction product of the step (I), and nicotinamide (NAM);
(III) purifying a reaction product of the step (II) with an ion-exchange column; and
(IV) applying a purification product of the step (III) to a dynamic axial compression column (DAC column).

Advantageous Effects of Invention

The present invention provides high-purity β-nicotinamide mononucleotide (NMN) having a purity of 99.0% by mass or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit. NMN highly purified to a level at which impurities are not detected, as in the high-purity βNMN of the present invention, has heretofore been absent. The high-purity βNMN of the present invention exerts an excellent anti-aging effect and also has high safety because the contents of impurities are equal to or less than a detection limit. Hence, the high-purity βNMN of the present invention is immune from adverse effects on the body even if taken over a long period. Furthermore, the high-purity βNMN of the present invention exerts an unexpected effect of being also superior in stability to conventional NMN. Thus, the high-purity βNMN of the present invention is suitably used in functional foods, medicaments, quasi-drugs, and the like for anti-aging purposes.

BRIEF DESCRIPTION OF DRAWINGS

[Fig. 1] Fig. 1 shows LC chromatograms of the high-purity βNMN of the present invention and two commercially available products of NMN.
[Fig. 2] Fig. 2 shows LC chromatograms (enlarged) of the high-purity βNMN of the present invention and two commercially available products of NMN.
[Fig. 3] Fig. 3 shows a mass spectrum and a product ion spectrum of component 7 (the high-purity βNMN of the present invention).
[Fig. 4] Fig. 4 shows a mass spectrum and a product ion spectrum of component 2 (commercially available product A).
[Fig. 5] Fig. 5 shows a mass spectrum and a product ion spectrum of component 5 (commercially available product A).
[Fig. 6] Fig. 6 shows a mass spectrum and a product ion spectrum of component 7 (commercially available product A).
[Fig. 7] Fig. 7 shows a mass spectrum and a product ion spectrum of component 1 (commercially available product B).
[Fig. 8] Fig. 8 shows a mass spectrum and a product ion spectrum of component 2 (commercially available product B).
[Fig. 9] Fig. 9 shows a mass spectrum and a product ion spectrum of component 3 (commercially available product B).
[Fig. 10] Fig. 10 shows a mass spectrum and a product ion spectrum of component 4 (commercially available product B).
[Fig. 11] Fig. 11 shows a mass spectrum and a product ion spectrum of component 6 (commercially available product B).
[Fig. 12] Fig. 12 shows a mass spectrum and a product ion spectrum of component 7 (commercially available product B).
[Fig. 13] Fig. 13 shows a mass spectrum and a product ion spectrum of component 8 (commercially available product B).

[Fig. 14] Fig. 14 shows a mass spectrum and a product ion spectrum of component 8 (commercially available product B).

DESCRIPTION OF EMBODIMENTS

Hereinafter, the present invention will be described in detail. The terms used herein are interpreted as having meanings usually used in the art, unless otherwise specified.

[High-purity β-nicotinamide mononucleotide (NMN)]

In the present invention, the nicotinamide mononucleotide (chemical formula: $C_{11}H_{15}N_2O_8P$) is a compound represented by a structural formula given below, is generally called NMN (nicotinamide mononucleotide), and is known as an intermediate metabolite involved in the biosynthesis of a coenzyme NAD+. In the living body, the nicotinamide mononucleotide is produced in a NAD metabolism pathway by liver tissues, i.e., a pathway from quinolinic acid after a kynurenine pathway toward the synthesis of nicotinamide adenine dinucleotide (NAD). In the living body, when tryptophan is used as a starting material, the tryptophan is converted to quinolinic acid (QA) through the kynurenine pathway which is a tryptophan metabolism pathway, and further to nicotinic acid mononucleotide (NaMN). On the other hand, when nicotinic acid (Na) is used as a starting material, the nicotinic acid is converted directly to NaMN. The NaMN is then converted to nicotinic acid adenine dinucleotide (NaAD) which is mutually converted to NAD, nicotinamide (NaM), and nicotinamide mononucleotide by a NAD cycle. The nicotinamide (NaM) is converted to nicotinamide mononucleotide by nicotinamide phosphoribosyltransferase (NAMPT). Subsequently, the nicotinamide mononucleotide is converted by nicotinamide mononucleotide adenyltransferase (NMNAT) to produce NAD. The nicotinamide mononucleotide is also produced from nicotinamide riboside (NR). The nicotinamide mononucleotide has two types of optical isomers, an α from and a β form. In the present invention, the β form having anti-aging action is used.

[Formula 1]

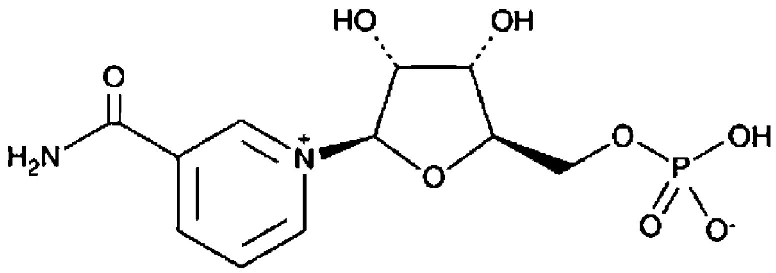

The high-purity β-nicotinamide mononucleotide (NMN) of the present invention has a purity of 99.0% by mass or more and has contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit. Although many supplements and the like containing NMN that claims a high purity have been commercially available so far, the NMN used as a starting material contains any of the impurities represented by the chemical formulas, in addition to the main component NMN, in all of these supplements and the like. In the high-purity βNMN of the present invention, the contents of the impurities represented by the chemical formulas are equal to or less than a detection limit.

The purity (%) of NMN is calculated by calculating the absolute content of the main component by an area normalization method based on LC-UV (high-performance liquid chromatography with ultraviolet absorption spectroscopy) or a quantitative NMR method (nuclear magnetic resonance spectroscopy).

In the area normalization method based on LC-UV, a NMN sample is dissolved in an appropriate amount of water and subjected to LC-UV measurement at a detection wavelength of 266 nm using an apparatus, for example, LC-30AD (manufactured by Shimadzu Corp.). The ratio (%) of the area of the main component to the total area of chromatogram peaks of the obtained sample can be regarded as the purity (%) of βNMN.

In the quantitative NMR method, a NMN sample and an internal standard are each dissolved in an appropriate amount of heavy water and subjected to NMR measurement under conditions of resonance frequency: $^1H$; 400 MHz, measurement mode: $^1H$ NMR, solvent: heavy water, internal standard (IS): dimethylsulfone, and reference substance: IS-derived signal; 3.16 ppm using, for example, FT-NMR apparatus JNM-ECA400 model (manufactured by JEOL RESONANCE Inc.). A value calculated according to the following expression from a $^1H$ NMR spectrum can be regarded as the purity (%).

$$P_a = S_a / S_s \times N_s / N_a \times M_a / M_s \times m_s / m_a \times P_s$$

$S_s$: integral value of the internal standard
$S_a$: integral value of the test substance
$N_s$: the number of protons in the internal standard (6)
$N_a$: the number of protons in the test substance (1)
$M_s$: molecular weight of the internal standard (94.13)
$M_a$: molecular weight of the test substance (334.22)
$m_s$: value of the internal standard weighed (mg)
$m_a$: value of the test substance weighed (mg)
$P_s$: purity (%) of the internal standard
$P_a$: purity of the test substance

The purity of the high-purity βNMN of the present invention is 99.0% by mass or more. The purity can be calculated as 99.0% by mass or more by any measurement method of the area normalization method based on LC-UV (high-performance liquid chromatography with ultraviolet absorption spectroscopy) and the quantitative NMR method (nuclear magnetic resonance spectroscopy) and is preferably calculated as 99.0% by mass or more by both the measurement methods.

The measurement and analysis of the impurities contained in NMN are performed by LC-MS and LC-MS/MS. The apparatus used or the like is not limited, and the measurement and the analysis can be performed at a detection wavelength of 266 nm in a measurement mass range m/z of 100 to 1500 using, for example, LC-30AD (manufactured by Shimadzu Corp.) or Q Exactive Plus (Thermo Fisher Scientific Inc.).

As a result of the measurement and the analysis, the contents of the substances represented by the chemical formulas of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ in the high-purity βNMN of the present invention are equal to or less than a detection limit. By sufficiently removing these impurities, the high-purity βNMN of the present invention can have a sufficiently high content of the main component NMN and have minimized contents of the impurities. These substances are considered as starting materials, intermediates, decomposition products, and the like in the production process of NMN. The chemical formulas that can be identified by the measurement and analysis method are as described above, and their respective putative structures are as given below. The number of each component corresponds to that shown in Table 3 in Examples.

$C_{11}H_{15}NO_9P$: component No. 2

[Formula 2]

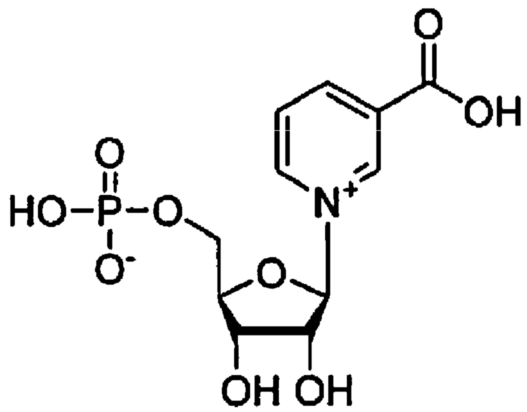

Chemical Formula: $C_{11}H_{14}NO_9P$
Exact Mass: 335.0406

$C_{10}H_{16}N_5O_{10}P_2$: component No. 5

[Formula 3]

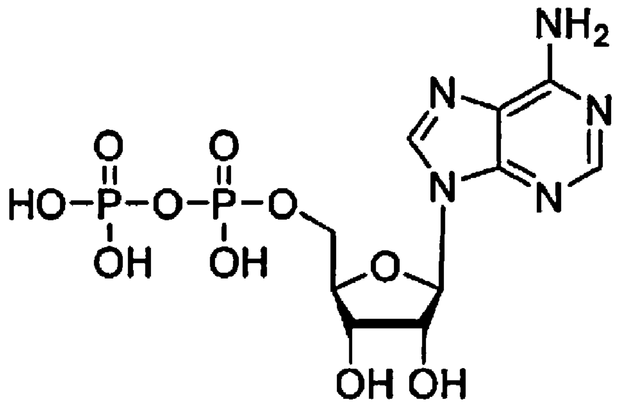

Chemical Formula: $C_{10}H_{15}N_5O_{10}P_2$
Exact Mass: 427.0294
adenosine diphosphate (ADP)

$C_9H_{15}N_3O_8P$: component No. 1

[Formula 4]

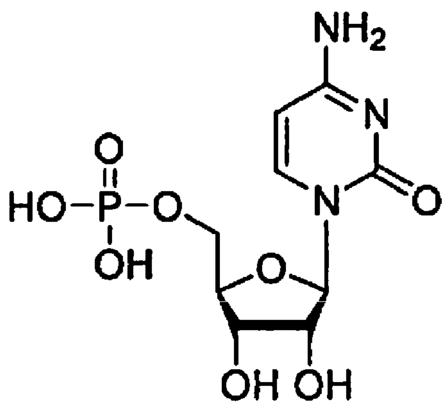

Chemical Formula: $C_9H_{14}N_3O_8P$
Exact Mass: 323.0519
cytidine monophosphate (CMP)

$C_9H_{15}N_3O_7P$: component No. 3

[Formula 5]

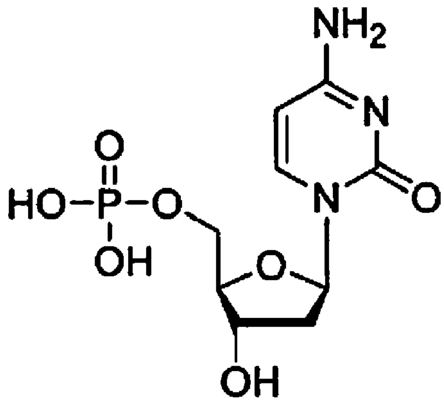

Chemical Formula: $C_9H_{14}N_3O_7P$
Exact Mass: 307.0569
deoxycytidine monophosphate (dCMP)

$C_9H_{16}N_4O_8P$: component No. 4

[Formula 6]

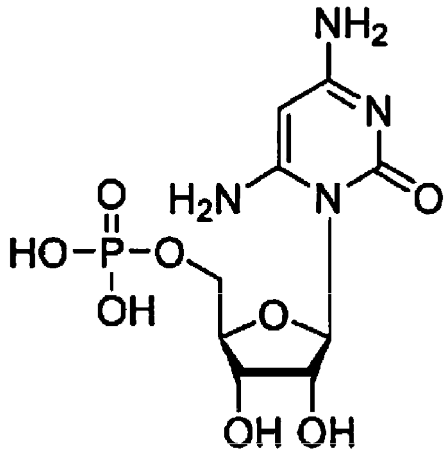

Chemical Formula: $C_9H_{15}N_4O_8P$
Exact Mass: 338.0627

$C_{10}H_{15}N_5O_7P$: component No. 6

[Formula 7]

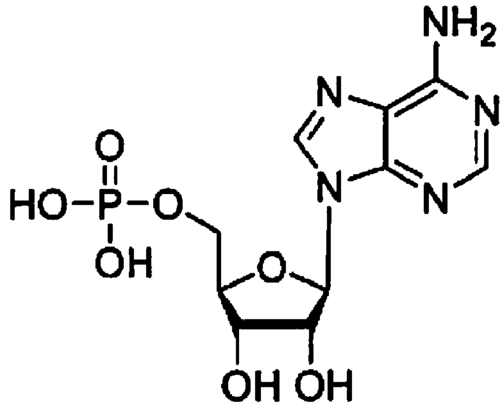

Chemical Formula: $C_{10}H_{14}N_5O_7P$
Exact Mass: 347.0631
adenosine monophosphate (AMP)

$C_{21}H_{27}N_6O_{15}P_2$: component No. 8

[Formula 8]

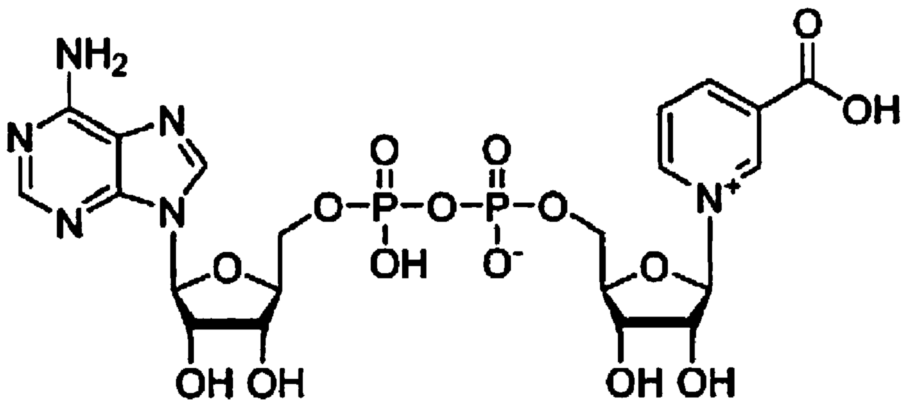

Chemical Formula: $C_{21}H_{26}N_6O_{15}P_2$
Exact Mass: 664.0931

The high-purity βNMN of the present invention is excellent in anti-aging action and is also expected to be effective for the prevention of a disease such as Alzheimer's disease or heart failure or the improvement of symptoms thereof. Such high-purity βNMN of the present invention is expected to be used as a component in functional foods, medicaments, cosmetics, and the like. The present invention also encompasses a pharmaceutical composition containing the high-purity β-nicotinamide mononucleotide (NMN) mentioned above as an active ingredient. The present invention also encompasses a composition for aging prevention containing the high-purity β-nicotinamide mononucleotide (NMN) mentioned above as an active ingredient.

The pharmaceutical composition or the composition for aging prevention of the present invention can be used in medicaments, quasi-drugs, foods, functional foods, and the like. Such a composition of the present invention may contain, in addition to the high-purity βNMN of the present invention mentioned above, other components depending on use and a form without impairing the advantageous effects of the present invention.

Examples of other components described above include pharmaceutically acceptable carriers and additives. Examples of such carriers and additives include, but are not limited to, water, tonicity adjusting agents, thickeners, sugars, sugar alcohols, antiseptics (preservatives), germicidal agents or antimicrobial agents, pH adjusters, stabilizers, chelating agents, oil bases, gel bases, surfactants, suspending agents, binders, excipients, lubricants, disintegrants, effervescent agents, fluidizers, dispersants, emulsifiers, buffers, solubilizers, antioxidants, sweeteners, acidulants, colorants, flavors, fragrances, and cooling agents.

The amount of the high-purity βNMN contained in the composition of the present invention can be appropriately adjusted depending on use and a form. The amount is usually 0.1% by weight to 100% by weight and can be 1% by weight to 95% by weight, 1% by weight to 90% by weight, 1% by weight to 85% by weight, 1% by weight to 80% by weight, or 2% by weight to 75% by weight. The amount is preferably 1% by weight to 85% by weight, more preferably 1% by weight to 80% by weight, further preferably 2% by weight to 75% by weight, in consideration of blend of the stability or handleability and effect of a preparation.

The composition of the present invention can be administered orally or parenterally and locally or systemically. Examples of the administration route include an oral route, an intravenous route, an intramuscular route, an intraarterial route, an intramedullary route, an intrathecal route, an intracerebroventricular route, a percutaneous route, a subcutaneous route, an intraperitoneal route, an intranasal route, a local route, a sublingual route, and a rectal route. Among them, an oral route, an intravenous route, an intramuscular route, and a subcutaneous route are listed as preferred administration routes.

The composition of the present invention can be prepared and formulated, for example, by mixing the high-purity βNMN of the present invention with a pharmaceutically acceptable carrier and/or an excipient, etc., by a routine method.

Examples of the dosage form of the composition of the present invention to be provided as a medicament or a quasi-drug include: oral agents such as tablets (including sugar-coated tablets), capsules, granules, powders, troches, chewables, pills, liquid medications, emulsions, syrups, suspensions, and elixirs; and external preparations such as ointments, gels, creams, and patches; and parenteral agents such as injections (e.g., subcutaneous injections, intravenous injections, intramuscular injections, and intraperitoneal injections), drops, and suppositories. The composition of the present invention to be provided as a food or a functional food may be in the form of a usual food or may be prepared as a supplement or the like in a form such as a capsule, granules, a powder, a troche, a chewable, a pill, a liquid medication, an emulsion, a syrup, a suspension, or an elixir.

The composition of the present invention can be suitably used for reduction in physical activity, reduction in quality of sleep, reduction in cognitive function, reduction in glucose metabolism, reduction in vision, type II diabetes mellitus, obesity, obesity associated with aging, increase in lipid level in blood associated with aging, reduction in insulin sensitivity associated with aging, loss or reduction of memory function associated with aging, loss or reduction of eye function associated with aging, reduction in physiological function associated with aging, impairment of glucose-stimulated insulin secretion, type I diabetes mellitus, improvement in mitochondrial function, neural death, Alzheimer's disease, cardiac ischemia, reperfusion injury, maintenance of neural stem cells and/or a neural progenitor cell population, recovery of mitochondrial function and arterial function in skeletal muscle after damage, muscle diseases (diseases involving muscle weakness, muscle atrophy, or loss of muscle strength, specifically, sarcopenia, dynapenia, cachexia, muscular dystrophy, myotonic disorder, spinal muscular atrophy, and myopathy), and the like.

The dose of the composition of the present invention needs to be determined depending on the circumstance of a patient in need of treatment or a subject in need of improvement. The dose and the number of doses can be adjusted so as to sufficiently exert the effects of the high-purity βNMN. In an embodiment of the present invention, the composition of the present invention is administered at a dose of 1 mg to 10 g, preferably 10 mg to 5 g, more preferably 50 mg to 1 g, further preferably 100 mg to 1 g, particularly preferably 200 mg to 600 mg, in terms of the amount of the high-purity βNMN per day.

The recipient of the composition of the present invention is a mammal and is preferably a human, a horse, a bovine, a dog, a cat, or the like, more preferably a human.

[Method for producing high-purity βNMN]

The present invention also encompasses a method for producing high-purity β-nicotinamide mononucleotide (NMN), comprising the steps of: (I) reacting AMP nucleosidase, ribose-phosphate diphosphokinase, adenosine monophosphate (AMP), adenosine triphosphate (ATP), and ribose-5-phosphate (R5P); (II) reacting nicotinamide phosphoribosyltransferase (Nampt), a reaction product of the step (I), and nicotinamide (NAM); (III) purifying a reaction product of the step (II) with an ion-exchange column; and (IV) applying a purification product of the step (III) to a dynamic axial compression column (DAC column). The production method of the present invention preferably further comprises the step of (V) crystallizing a purification product of the step (IV). The method of the present invention can produce the high-purity βNMN having a purity of 99.0% by mass or more and having contents of $C_{11}H_{15}NO_9P$, $C_{10}H_{16}N_5O_{10}P_2$,

$C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit as mentioned above.

Hereinafter, the method for producing high-purity βNMN according to the present invention will be described in detail with reference to an embodiment in which each step is performed. However, the production method of the present invention may be performed by an appropriately changed or modified embodiment of each step specifically described below, without departing from the spirit of the present invention.

<Step (I)>

This step is the step of reacting AMP nucleosidase, ribose-phosphate diphosphokinase, adenosine monophosphate (AMP), adenosine triphosphate (ATP), and ribose-5-phosphate (R5P). Phosphoribosyl pyrophosphate (PRPP), a reaction product of this step, is used in the reaction of the step (II).

(AMP nucleosidase)

The AMP nucleosidase (EC3.2.2.4) is an enzyme that catalyzes chemical reaction given below. This enzyme is classified into a hydrolytic enzyme, particularly, glycosidase that decomposes a N-glycosyl compound. Its systemic name is AMP phosphoribohydrolase. This enzyme is also called adenylate nucleosidase or the like.

[Formula 9] Adenosine monophosphate + Water ⇄ D-Ribose-5-phosphate + Adenine

(Ribose-phosphate diphosphokinase)

The ribose-phosphate diphosphokinase (EC2.7.6.1) is also called ribose phosphate pyrophosphokinase or phosphoribosyl pyrophosphate synthase. In addition, this enzyme is called PPRibP synthetase; PP-ribose P synthetase; 5-phosphoribosyl-1-pyrophosphate synthetase; 5-phosphoribose pyrophosphorylase; 5-phosphoribosyl-alpha-1-pyrophosphate synthetase; phosphoribosyl-diphosphate synthetase; phosphoribosylpyrophosphate synthase; pyrophosphoribosylphosphate synthetase; ribophosphate pyrophosphokinase; ribose-5-phosphate pyrophosphokinase, or the like. This enzyme catalyzes chemical reaction given below. The enzyme is involved in the synthesis of nucleotides having purine or pyrimidine, cofactors NAD and NADP, and amino acids such as histidine and tryptophan. The enzyme applies ribose-5-phosphate derived from a pentose phosphate pathway to synthesis systems thereof.

[Formula 10] ATP + D-Ribose 5-phosphate ⇄ AMP + 5-Phospho-o-D-ribose 1-diphosphate

The enzymes for use in this step may be natural enzymes or may be variant enzymes, preferably improved in expression level or enzymatic activity, prepared by engineering the amino acid sequences of the natural enzymes as long as the enzymes can catalyze their respective reactions of interest. For the purpose of, for example, simplifying purification, promoting soluble expression, or performing detection with an antibody, various tags (proteins or peptides) may be added to each enzyme. Examples of the types of the tags include His tag (histidine tag, Strep(II)-tag, GST tag (glutathione-S-transferase tag), MBP tag (maltose binding protein tag), GFP tag (green fluorescent protein tag), SUMO tag (small ubiquitin-related (like) modifier tag) FLAG tag, HA tag, and myc tag. The enzymes may be expressed as fusion proteins with each other and used.

The enzymes for use in this step are preferably immobilized on carriers or the like in order to improve reaction efficiency. The immobilization method is not particularly limited and can be performed, for example, by the following method.

The ribose-phosphate diphosphokinase (ribose phosphate pyrophosphokinase) and the AMP nucleosidase are each diluted with an enzyme washing buffer (0.02 M Tris-HCl/0.001 M EDTA solution, pH 7.0) into a protein content of 5 to 10 mg/ml. Each enzyme dilution and a PB solution (2.0 mol/L potassium dihydrogen phosphate, pH 7.5) were mixed in equal amounts. An enzyme immobilization carrier such as Epoxy LX-3000 model (50 mg of an enzyme/1 g of a carrier) is added thereto and reacted at 25°C for 20 hours using a shaker (at a rotational speed of 150 rpm). After the completion of reaction, the reaction solution can be filtered through a filter bag and washed 5 or 6 times with an enzyme washing buffer to obtain immobilized ribose-phosphate diphosphokinase (ribose phosphate pyrophosphokinase) and immobilized AMP nucleosidase.

One example of a specific form of this step is as follows: a substrate solution containing 0.1 mM to 200 mM ATP, 0.1 mM to 300 mM AMP, 0.1 mM to 100 mM MgCla, 0.1 mM to 100 mM KCl, and a 10 to 200 mM Tris-HCl buffer solution is provided, added to a reaction vessel, and adjusted to pH 6.5 to 8.5, preferably pH 7.0 to 7.5. The AMP nucleosidase and the ribose-phosphate diphosphokinase are both added at 0.1 g/L to 200 g/L thereto and reacted by

stirring at a speed of 50 rpm under a condition of 30 to 50°C, preferably 35°C to 40°C, more preferably 37°C. One to 8 hours later, the reaction is terminated, and the process proceeds to the II step.

<Step (II)>

This step is the step of reacting nicotinamide phosphoribosyltransferase (Nampt), a reaction product of the step (I), and nicotinamide (NAM). This step produces a crude product solution of NMN.

(Nicotinamide phosphoribosyltransferase (Nampt))

The Nampt (EC2.4.2.12) is generally known to participate in a NAD (nicotinamide adenine dinucleotide) salvage pathway. In the present invention, this enzyme is used for reaction that produces NMN from PRPP and NAM. In the NMN synthesis reaction from PRPP and NAM by the Nampt, ATP is originally not essential. However, ATP may be added in this step because the Nampt has ATP hydrolytic activity and the autophosphorylation of the Nampt by the hydrolysis of ATP changes enzymological parameters or chemical equilibrium in NMN production's favor.

Examples of the Nampt include a human *(Homo sapiens)-derived* one (NP_005737), a mouse (*Mus musculus*)-*derived* one (NP_067499), a rat (*Rattus norvegicus*)-*derived* one (NP_808789), a zebrafish (*Danio* rerio)-derived one (NP_997833), and ones derived from bacteria such as *Haemophilus ducreyi* (AAR87771), *Deinococcus radiodurans* (AE001890), *Oenococcus oeni* (KZD13878), and *Shewanella oneidensis* (NP_717588). In this step, bacterium-derived Nampt is preferably used. In this context, the bacteria are a group of prokaryotes having no nuclear membrane and are an organism group including *Escherichia coli, Bacillus subtilis,* and *Cyanobacteria.*

The Nampt for use in this step may be a natural enzyme or may be a variant enzyme, preferably improved in expression level or enzymatic activity, prepared by engineering the amino acid sequence of the natural enzyme as long as the enzyme can catalyze the reaction of interest. For the purpose of, for example, simplifying purification, promoting soluble expression, or performing detection with an antibody, various tags (proteins or peptides) may be added to each enzyme. The types of the tags are as described in the step (I).

The Nampt for use in this step is preferably immobilized on a carrier or the like in order to improve reaction efficiency. The immobilization method is as described in the step (I).

One example of a specific form of this step is as follows: the reaction solution obtained in the step (I) is transferred to another reaction vessel. Nicotinamide (NAM), MgCla, and a Tris-HCl buffer solution are added thereto such that their final concentrations are 1 to 100 mM, 1 mM to 100 mM, and 20 to 100 mM, respectively. Further, the Nampt is added thereto at 1 to 100 g/L and reacted for 1 to 8 hours with stirring at a speed of 50 rpm at 30°C to 50°C, preferably 35°C to 40°C, more preferably 37°C, at pH maintained on the order of 6, i.e., under a condition of pH 6.5 to 8.5, preferably pH 7.5 to 8.0. The obtained reaction solution is filtered, and the process proceeds to the step (III). This step produces a crude product of NMN.

<Step (III)>

This step is the step of purifying a reaction product of the step (II) with an ion-exchange column. This step produces NMN having a high purity from the crude product of NMN obtained in the step (II).

One example of a specific form of this step is as follows: the filtrate obtained in the step (II) is applied to an ion-exchange column (DEAE-Toyopearl, MonoQ column, etc.) to recover a purified solution. The nanofiltered sample is subjected to the step (IV).

<Step (IV)>

This step is the step of applying a purification product of the step (III) to a dynamic axial compression column (DAC column) for high-pressure separation. This step sufficiently removes the impurities and produces high-purity βNMN having a purity of 99.0% or more.

The dynamic axial compression column (DAC column) used in the present invention is not particularly limited, and a generally commercially available dynamic axial compression column (DAC column) can be used. One example of such an apparatus includes DAC300 (Beijing Tong Heng Innovation Technology Co., Ltd.). The column is packed with an anion-exchange resin or a cation-exchange resin and used.

The high-purity βNMN obtained by the high-pressure separation is decolorized by application to a column for decolorization or the like and further subjected to nanofiltration and concentration, and the obtained purification product is subjected to the next step (V).

<Step (V)>

This step is the step of crystallizing a purification product of the step (IV). The solvent for use in crystallization is preferably ethanol. For further enhancing the purity and removing the impurities, recrystallization is preferably performed by the same method as above. The purification product obtained by this step is high-purity βNMN having a purity of 99.0% or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit.

EXAMPLES

Hereinafter, the present invention will be described in more detail with reference to Examples. However, the present invention is not limited by these examples by any means.

1. Production of high-purity βNMN

(1) Immobilization of enzyme

Nampt, ribose-phosphate diphosphokinase (ribose phosphate pyrophosphokinase) and AMP nucleosidase were each diluted with an enzyme washing buffer (0.02 M Tris-HCl/0.001 M EDTA solution, pH 7.0) into a protein content of 5 to 10 mg/ml. Each enzyme dilution and a PB solution (2.0 mol/L potassium dihydrogen phosphate, pH 7.5) were mixed in equal amounts. An enzyme immobilization carrier such as Epoxy LX-3000 model (50 mg of an enzyme/1 g of a carrier) was added thereto and reacted at 25°C for 20 hours using a shaker (at a rotational speed of 150 rpm). After the completion of reaction, the reaction solution was filtered through a filter bag and washed 5 or 6 times with an enzyme washing buffer to obtain immobilized Nampt, immobilized ribose-phosphate diphosphokinase (ribose phosphate pyrophosphokinase) and immobilized AMP nucleosidase.

(2) I step

A substrate solution containing 15 mM ATP, 100 mM AMP, 10 mM MgCla, 10 mM KCl, and a 70 mM Tris-HCl buffer solution was provided, added to a reaction vessel, and adjusted to pH 7.0 to 7.5. The AMP nucleosidase and the ribose-phosphate diphosphokinase were both added at 20 g/L thereto and reacted by stirring at a speed of 50 rpm under a condition of 35°C. Three hours later, the reaction was terminated, and the process proceeded to the II step.

(3) II step

The reaction solution obtained in the I step was transferred to another reaction vessel. Nicotinamide (NAM), MgCla, and a Tris-HCl buffer solution were added thereto such that their final concentrations were 60 mM, 20 mM, and 100 mM, respectively. Further, the Nampt was added thereto at 20 g/L and reacted by stirring at a speed of 50 rpm at 35°C under a condition of pH 7.5 to 8.0. Three hours later, the reaction was terminated, and the reaction solution was filtered, and the process proceeded to the III step.

(4) III step

The filtrate obtained in the II step was applied to an ion-exchange column (DEAE-Toyopearl, MonoQ column, etc.) to recover a purified solution.

(5) IV step

The purified solution obtained in the III step was nanofiltered for concentration to recover a concentrated solution. The recovered concentrated solution was applied to a dynamic axial compression column DAC-300 (Beijing Tong Heng Innovation Technology Co., Ltd.) to remove impurities. Further, a decolorized solution was obtained by decolorization with a resin. Then, the decolorized solution was concentrated by nanofiltration.

(6) V step

The purification product obtained in the IV step was crystallized with ethanol to obtain a crude product. The crude product was redissolved, recrystallized with ethanol, and freeze-dried to obtain a powder of the high-purity βNMN of the present invention.

2. Characteristics of high-purity βNMN

(1) Purity of high-purity βNMN

The absolute content of the main component was calculated by the area normalization method based on LC-UV (high-performance liquid chromatography with ultraviolet absorption spectroscopy) and the quantitative NMR method (nuclear magnetic resonance spectroscopy).

In the area normalization method based on LC-UV, the high-purity βNMN obtained in the section (5) was dissolved in an appropriate amount of water and subjected to LC-UV measurement under the following conditions.

Apparatus name: LC-30AD (manufactured by Shimadzu Corp.)
Detection wavelength: 266 nm

The ratio (%) of the area of the main component to the total area of chromatogram peaks of the obtained sample was regarded as the purity (%) of βNMN. As a result, the purity of the high-purity βNMN obtained in the section (5) was 99.2%.

In the quantitative NMR method, the high-purity βNMN obtained in the section (5) and an internal standard were each dissolved in an appropriate amount of heavy water and subjected to NMR measurement under the following conditions.

Apparatus name: FT-NMR apparatus JNM-ECA400 model (manufactured by JEOL RESONANCE Inc.)
Resonance frequency: $^{1}H$; 400 MHz
Measurement mode: $^{1}H$ NMR
Solvent: heavy water
Internal standard (IS): dimethylsulfone
Reference substance: IS-derived signal; 3.16 ppm

A value calculated according to the following expression from a $^{1}H$ NMR spectrum was regarded as the purity (%). As a result, the purity of the high-purity βNMN obtained in the section (5) was 99.4%.

$$P_a = S_a / S_s \times N_s / N_a \times M_a / M_s \times m_s / m_a \times P_s$$

$S_s$: integral value of the internal standard
$S_a$: integral value of the test substance
$N_s$: the number of protons in the internal standard (6)
$N_a$: the number of protons in the test substance (1)
$M_s$: molecular weight of the internal standard (94.13)
$M_a$: molecular weight of the test substance (334.22)
$m_s$: value of the internal standard weighed (mg)
$m_a$: value of the test substance weighed (mg)
$P_s$: purity (%) of the internal standard
$P_a$: purity of the test substance

(2) Stability

The high-purity βNMN obtained in the section (5) was preserved at 4°C, 25°C, or 40°C and examined for its stability. Each sample was provided as three samples per condition. The purity of βNMN in each sample during preservation, 1 month later, 2 months later, and 3 months later was measured by the area normalization method based on LC-UV. The results are shown in Table 1 below.

[Table 1]

| Stability evaluation | | Purity (%) | |
|---|---|---|---|
| | | Average value (%) | S.D. |
| 4°C | During preservation | 99.69 | 0.25 |
| | 1 month later | 99.64 | 0.16 |
| | 2 months later | 99.51 | 0.03 |
| | 3 months later | 99.66 | 0.21 |
| 25°C | During preservation | 99.69 | 0.25 |
| | 1 month later | 99.26 | 0.56 |
| | 2 months later | 99.45 | 0.09 |
| | 3 months later | 99.57 | 0.16 |
| 40°C | During preservation | 99.69 | 0.25 |
| | 1 month later | 99.51 | 0.19 |
| | 2 months later | 99.27 | 0.11 |
| | 3 months later | 99.35 | 0.26 |

As shown in Table 1, the high-purity βNMN obtained in the section (5) was able to maintain the purity of 99% or more for 3 months when preserved at any temperature of 4°C, 25°C, and 40°C. This result indicates that the high-purity βNMN obtained in the section (5) is excellent in stability.

Results of a stability test on NMN produced by a conventional method (method involving neither the step IV nor the step V according to the present invention) are shown as Comparative Examples in Table 2.

[Table 2]

| Stability evaluation | Purity (%) | |
|---|---|---|
| | 4°C | 25°C |
| At start of preservation | 97.94 | 97.94 |
| 1 day later | 97.92 | 97.11 |
| 2 days later | 97.78 | 96.04 |
| 3 days later | 97.60 | 95.98 |
| 7 days later | 97.57 | 90.55 |
| 15 days later | 97.54 | 81.74 |
| 30 days later | 97.26 | 65.66 |

As shown in Table 2, the NMN produced by the conventional method was able to maintain the purity obtained at the start of preservation for 30 days when preserved at 4°C, and however, when preserved at 25°C (room temperature), exhibited reduction in purity over time which was, albeit 97.94% at the start of preservation, 81.74% 15 days later and 65.66% 30 days later. From this result, the NMN obtained by the production method of the present invention was found to be able to maintain the high purity over a long period as compared with the NMN produced by the conventional method, and to be excellent in stability.

(3) Impurity content

The contents of impurities were measured as to the high-purity βNMN of the present invention obtained in the section (5), commercially available product A of NMN, and commercially available product B of NMN. The main component (NMN) and the impurities contained in these NMN products were measured by LC-MS and LC-MS/MS. The analysis conditions are as follows.

Apparatus name: LC-30AD (manufactured by Shimadzu Corp.)
Detection wavelength: 266 nm
MS apparatus: Q Exactive Plus (Thermo Fisher Scientific Inc.)
Measurement mass range: m/z 100-1500

The obtained chromatograms are shown in Fig. 1. The impurities to be analyzed were numbered from components having a shorter retention time in order (Fig. 2). A common component between the samples was given the same number (component Nos. 1 to 8). The mass spectrum and product ion spectrum of each peak of each sample are shown in Figs. 3 to 14. Results about the accurate mass and compositional operation of each component (component Nos. 1 to 8) are shown in Table 3. N.D. in the table represents that the content of the component was equal to or less than a detection limit.

[Table 3]

| Component No. | High-purity βNMN of present invention | Commercially available product A | Commercially available product B |
|---|---|---|---|
| 1 | N.D. | N.D. | 324.0585 $[M+H]^+$ $C_9H_{15}N_3O_8P$ |
| 2 | N.D. | 336.0471 $[M+H]^+$ $C_{11}H_{15}NO_9P$ | 336.0469 $[M+H]^+$ $C_{11}H_{15}NO_9P$ |
| 3 | N.D. | N.D. | 308.0635 $[M+H]^+$ $C_9H_{15}N_3O_7P$ |
| 4 | N.D. | N.D. | 339.0695 $[M+H]^+$ $C_9H_{16}N_4O_8P$ |
| 5 | N.D. | 428.0367 $[M+H]^+$ $C_{10}H_{16}N_5O_{10}P_2$ | N.D. |
| 6 | N.D. | N.D. | 348.0701 $[M+H]^+$ $C_{10}H_{15}N_5O_7P$ |
| 7 | 123.0552 $[M+H]^+$ $C_6H_7N_2O$ | 123.0553 $[M+H]^+$ $C_6H_7N_2O$ | 123.0553 $[M+H]^+$ $C_6H_7N_2O$ |
| 8 | N.D. | N.D. | 665.1016 $[M+H]^+$ $C_{21}H_{27}N_8O_{15}P_2$ |

In the high-purity βNMN of the present invention, nicotinamide (NAM) of No. 7 was detected as a component other than the main component (NMN), whereas the contents of the other impurities were equal to or less than a detection limit. On the other hand, No. 2, No. 5, and No. 7 were detected in the commercially available product A, and No. 1, No. 2, No. 3, No. 4, No. 6, No. 7, and No. 8 were detected in the commercially available product B.

The absolute content of the main component was calculated as to the high-purity βNMN of the present invention, the commercially available product A of NMN, and the commercially available product B of NMN by the area normalization method based on LC-UV and the quantitative NMR method in the same manner as above. The results are shown in Table 4 below.

[Table 4]

| NMN sample | LC-UV Analysis | NMR Analysis |
|---|---|---|
| | Purity (%) | Purity (%) |
| High-purity βNMN of present invention | 99.0 | 99.4 |
| Commercially available product A | 98.3 | 94.4 |
| Commercially available product B | 97.5 | 99.0 |

The high-purity βNMN of the present invention had a purity of 99.0% or more measured by any method of the area normalization method based on LC-UV and the quantitative NMR method, and the contents of the impurities of Nos. 1 to 6 and 8 described in Table 3 above were equal to or less than a detection limit. Thus, the high-purity βNMN of the present invention had a high purity and had contents of the specific impurities equal to or less than a detection limit, suggesting that the high-purity βNMN of the present invention has high stability of the main component, maintains the high purity over a long period not only under a low-temperature condition but a room-temperature condition and under temperature conditions higher than room temperature, and is excellent in stability.

INDUSTRIAL APPLICABILITY

The present invention provides high-purity β-nicotinamide mononucleotide (NMN) having a purity of 99.0% by mass or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit. NMN highly purified to a level at which impurities are not detected, as in the high-purity βNMN of the present invention, has heretofore been absent. The high-purity βNMN of the present invention exerts an excellent anti-aging effect and also has high safety because the contents of impurities are equal to or less than a detection limit. Hence, the high-purity βNMN of the present invention is immune from adverse effects on the body even if taken over a long period. Furthermore, the high-purity βNMN of the present invention exerts an unexpected effect of being also superior in stability to conventional NMN. Thus, the high-purity βNMN of the present invention is suitably used in functional foods, medicaments, quasi-drugs, and the like for anti-aging purposes.

## Claims

1. High-purity β-nicotinamide mononucleotide (NMN) having a purity of 99.0% by mass or more and having contents of $C_{11}H_{15}NO_9P$, $C_{10}H_{16}N_5O_{10}P_2$, $C_9H_{15}N_3O_8P$, $C_9H_{15}N_3O_7P$, $C_9H_{16}N_4O_8P$, $C_{10}H_{15}N_5O_7P$, and $C_{21}H_{27}N_6O_{15}P_2$ as impurities equal to or less than a detection limit.

2. A pharmaceutical composition comprising high-purity β-nicotinamide mononucleotide (NMN) according to claim 1 as an active ingredient.

3. A composition for aging prevention comprising high-purity β-nicotinamide mononucleotide (NMN) according to claim 1 as an active ingredient.

4. A method for producing high-purity β-nicotinamide mononucleotide (NMN), comprising the steps of:

   (I) reacting AMP nucleosidase, ribose-phosphate diphosphokinase, adenosine monophosphate (AMP), adenosine triphosphate (ATP), and ribose-5-phosphate (R5P);
   (II) reacting nicotinamide phosphoribosyltransferase (Nampt), a reaction product of the step (I), and nicotinamide (NAM);
   (III) purifying a reaction product of the step (II) with an ion-exchange column; and
   (IV) applying a purification product of the step (III) to a dynamic axial compression column (DAC column).

[Fig. 1]

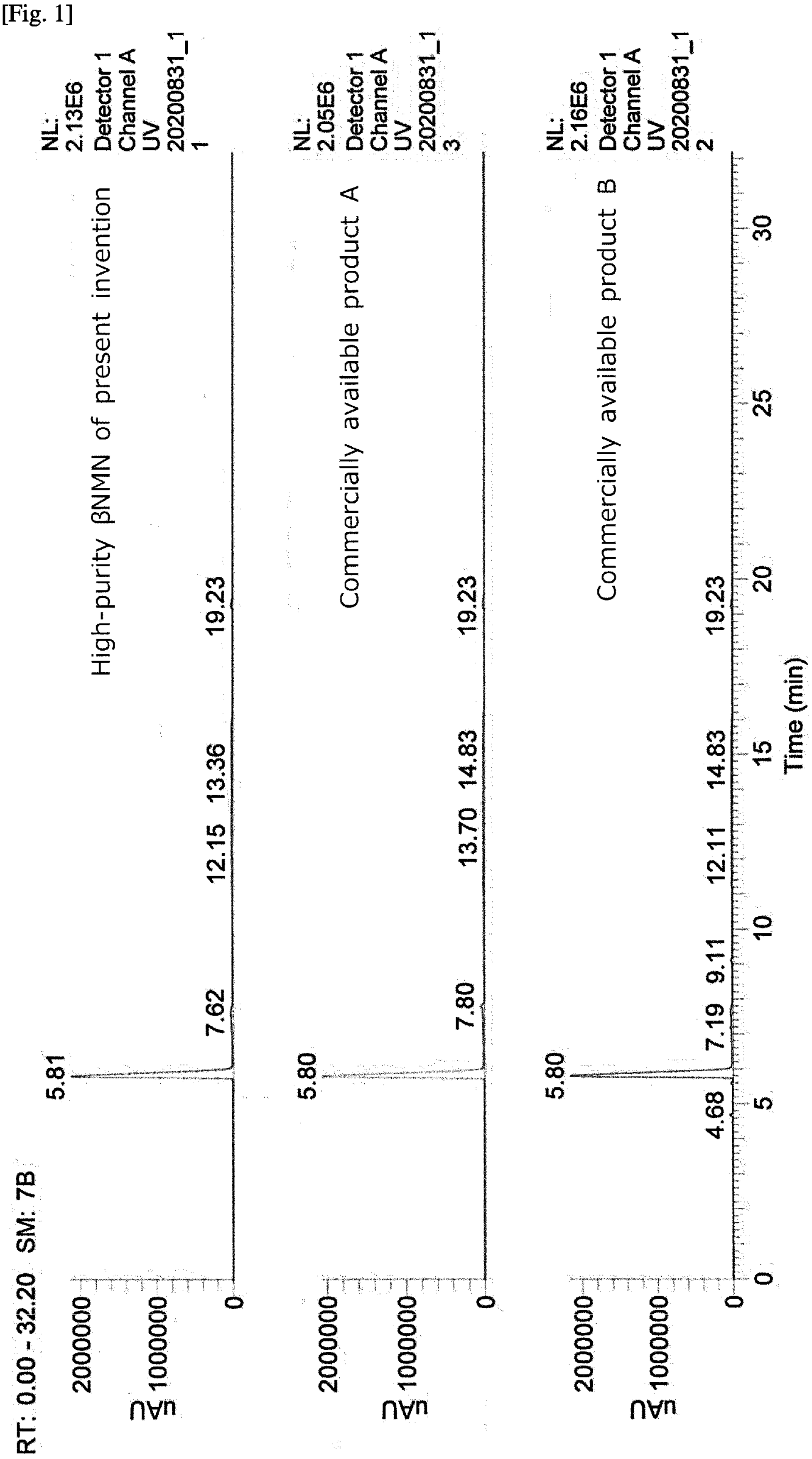
RT: 0.00 - 32.20 SM: 7B
High-purity βNMN of present invention
NL: 2.13E6 Detector 1 Channel A UV 20200831_11
uAU
2000000
1000000
0
5.81
7.62
12.15
13.36
19.23
Commercially available product A
NL: 2.05E6 Detector 1 Channel A UV 20200831_13
5.80
7.80
13.70
14.83
19.23
Commercially available product B
NL: 2.16E6 Detector 1 Channel A UV 20200831_12
4.68
5.80
7.19
9.11
12.11
14.83
19.23
0
5
10
15
20
25
30
Time (min)

[Fig. 2]

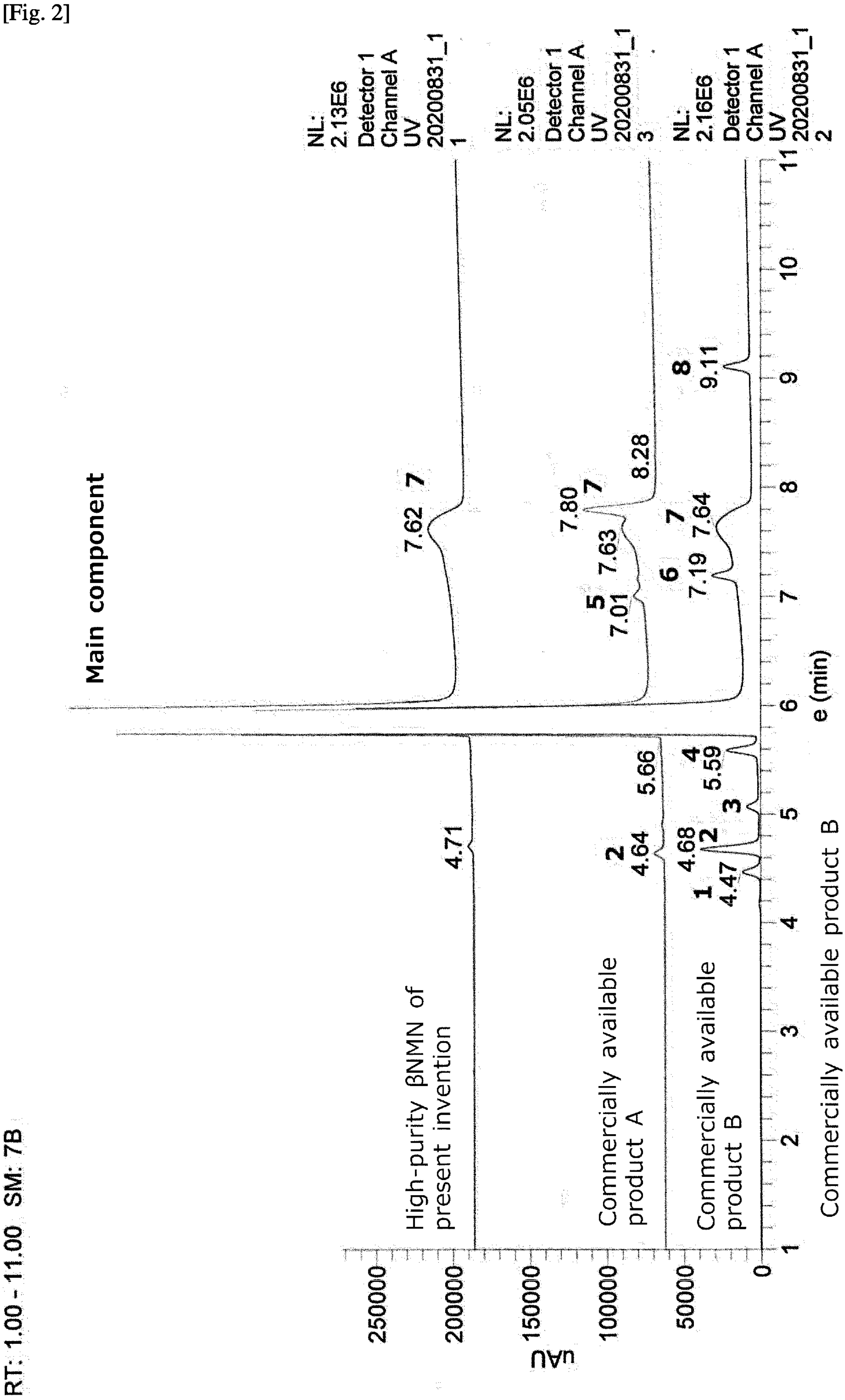
RT: 1.00 - 11.00 SM: 7B
uAU
250000
200000
150000
100000
50000
0
1
2
3
4
5
6
7
8
9
10
11
e (min)
Main component
High-purity βNMN of present invention
Commercially available product A
Commercially available product B
Commercially available product B
4.71
7.62
7
2
4.64
5.66
5
7.01
7.63
7.80
7
8.28
1
4.47
4.68
2
3
5.59
4
6
7.19
7
7.64
8
9.11
NL: 2.13E6 Detector 1 Channel A UV 20200831_1 1
NL: 2.05E6 Detector 1 Channel A UV 20200831_1 3
NL: 2.16E6 Detector 1 Channel A UV 20200831_1 2

[Fig. 3]

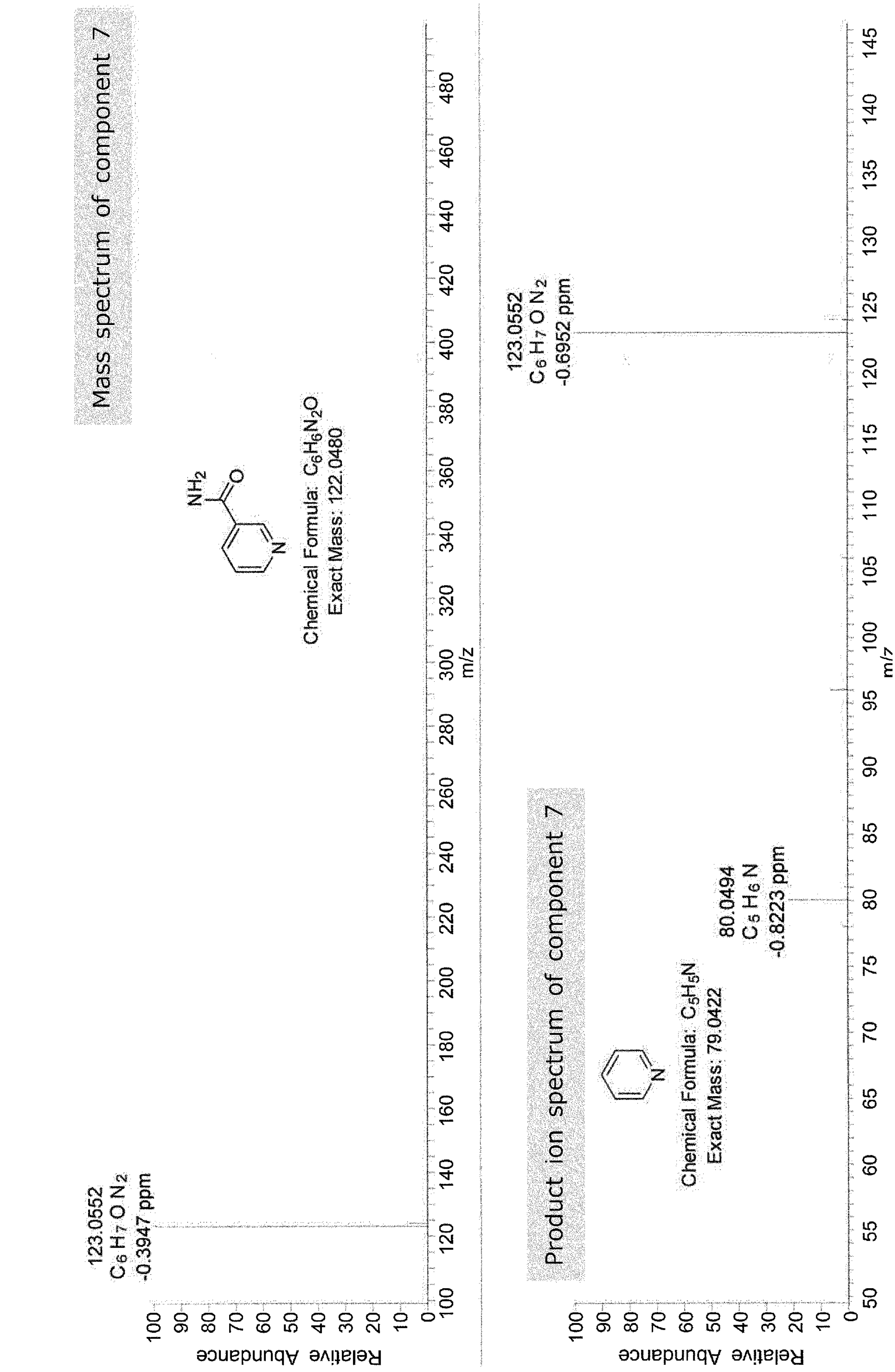
Mass spectrum of component 7
123.0552
C6 H7 O N2
-0.3947 ppm
Chemical Formula: C6H6N2O
Exact Mass: 122.0480
Relative Abundance
m/z
Product ion spectrum of component 7
123.0552
C6 H7 O N2
-0.6952 ppm
80.0494
C5 H6 N
-0.8223 ppm
Chemical Formula: C5H5N
Exact Mass: 79.0422
Relative Abundance
m/z

[Fig. 4]

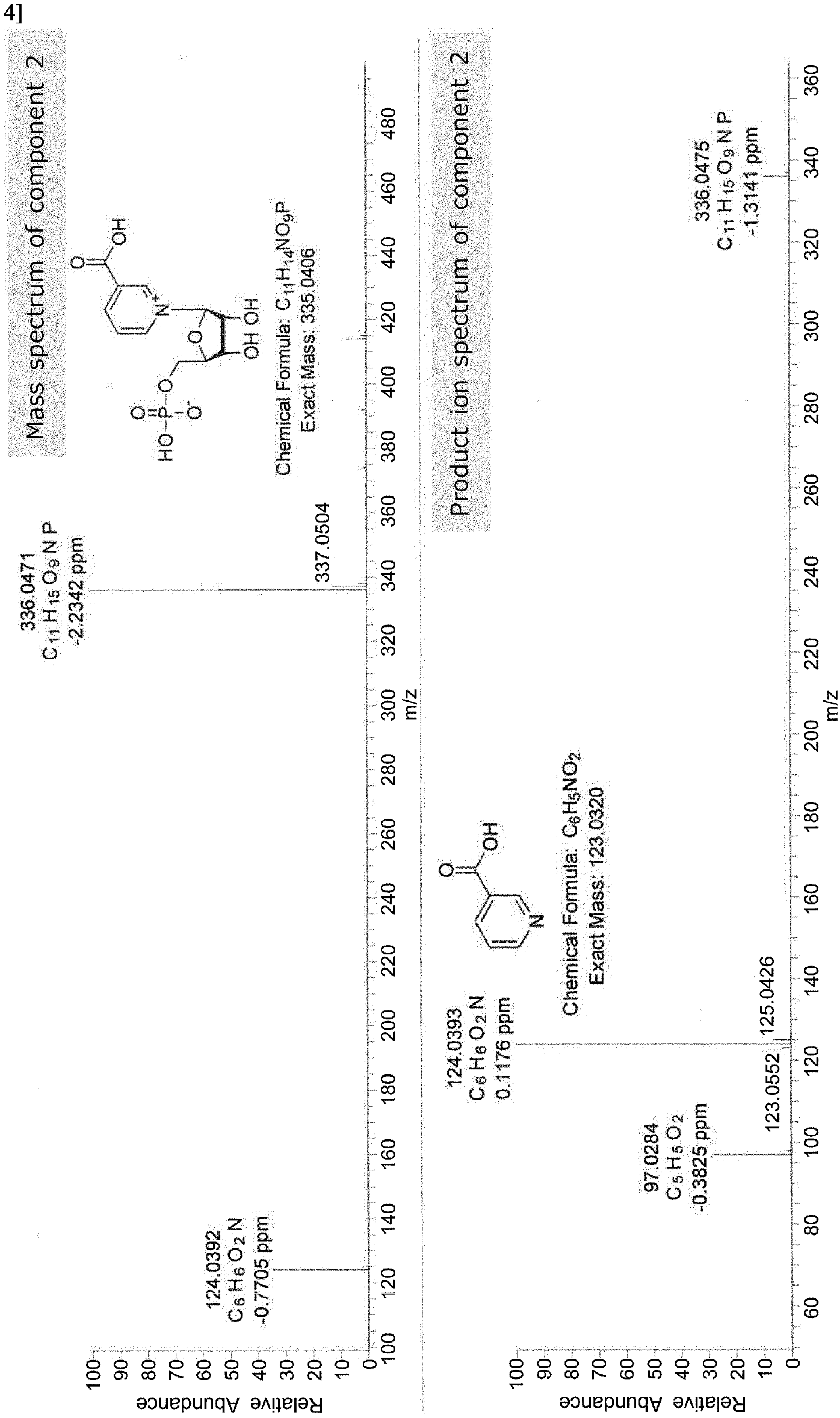
Mass spectrum of component 2
336.0471
C 11 H 15 O 9 N P
-2.2342 ppm
337.0504
124.0392
C 6 H 6 O 2 N
-0.7705 ppm
Chemical Formula: C11H14NO9P
Exact Mass: 335.0406
Relative Abundance
m/z
Product ion spectrum of component 2
336.0475
C 11 H 15 O 9 N P
-1.3141 ppm
124.0393
C 6 H 6 O 2 N
0.1176 ppm
125.0426
123.0552
97.0284
C 5 H 5 O 2
-0.3825 ppm
Chemical Formula: C6H5NO2
Exact Mass: 123.0320
Relative Abundance
m/z

[Fig. 5]

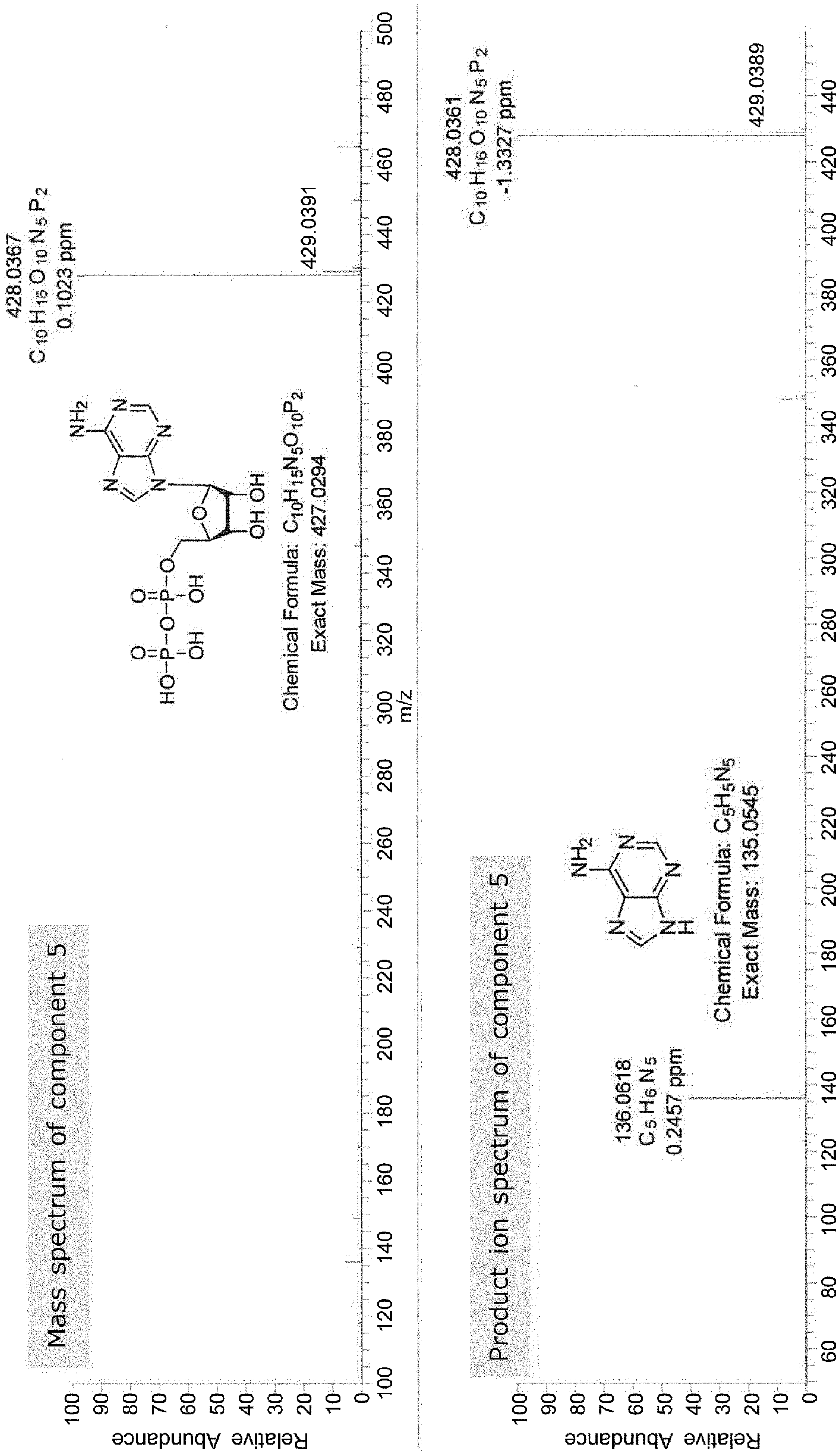

Mass spectrum of component 5
428.0367
$C_{10}H_{16}O_{10}N_5P_2$
0.1023 ppm
429.0391
Chemical Formula: $C_{10}H_{15}N_5O_{10}P_2$
Exact Mass: 427.0294
Relative Abundance
m/z
Product ion spectrum of component 5
136.0618
$C_5H_6N_5$
0.2457 ppm
Chemical Formula: $C_5H_5N_5$
Exact Mass: 135.0545
428.0361
$C_{10}H_{16}O_{10}N_5P_2$
-1.3327 ppm
429.0389
Relative Abundance
m/z

[Fig. 6]

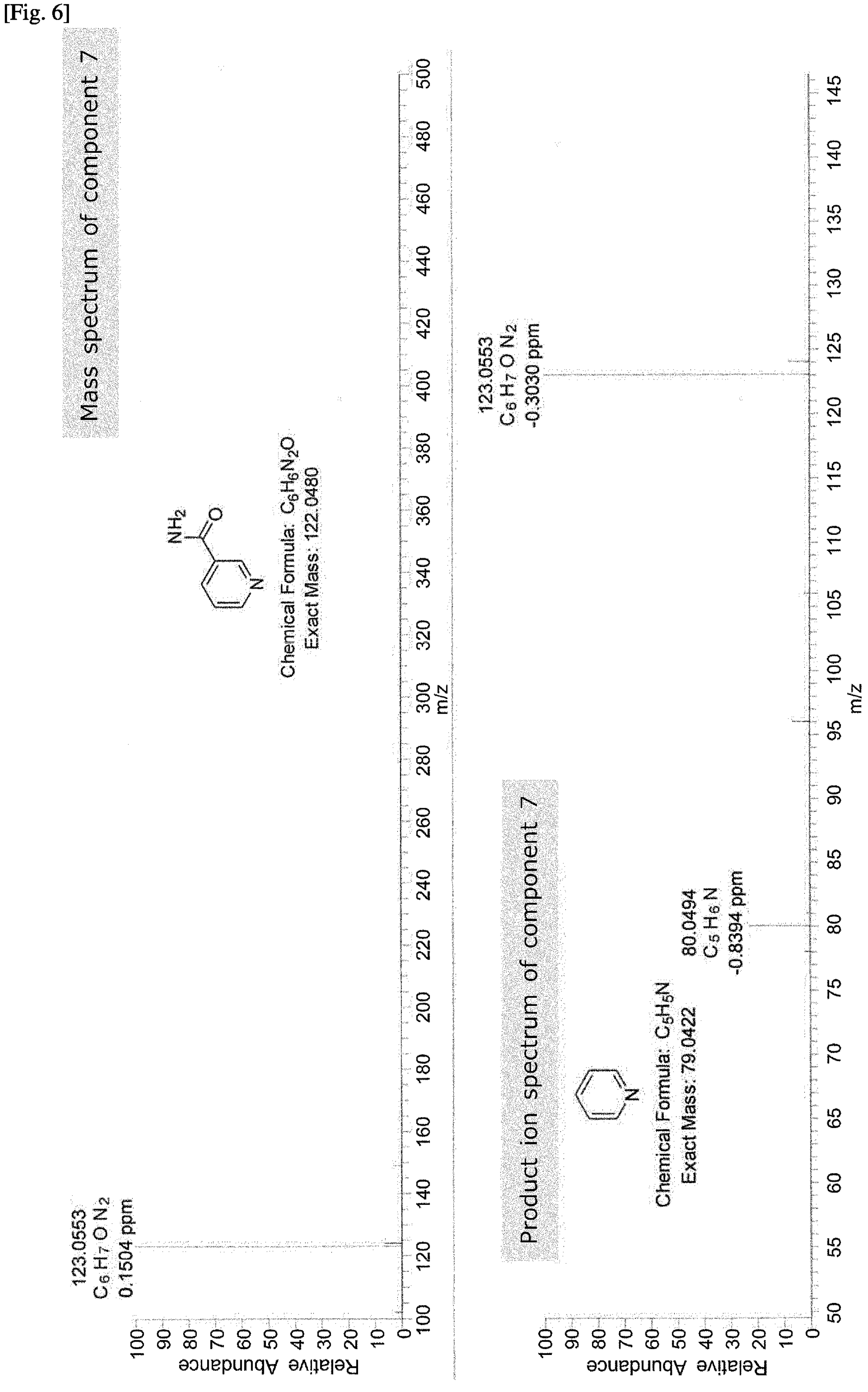
Mass spectrum of component 7
123.0553
C6 H7 O N2
0.1504 ppm
Chemical Formula: C6H6N2O
Exact Mass: 122.0480
Relative Abundance
m/z
Product ion spectrum of component 7
123.0553
C6 H7 O N2
-0.3030 ppm
80.0494
C5 H6 N
-0.8394 ppm
Chemical Formula: C5H5N
Exact Mass: 79.0422
Relative Abundance
m/z

[Fig. 7]

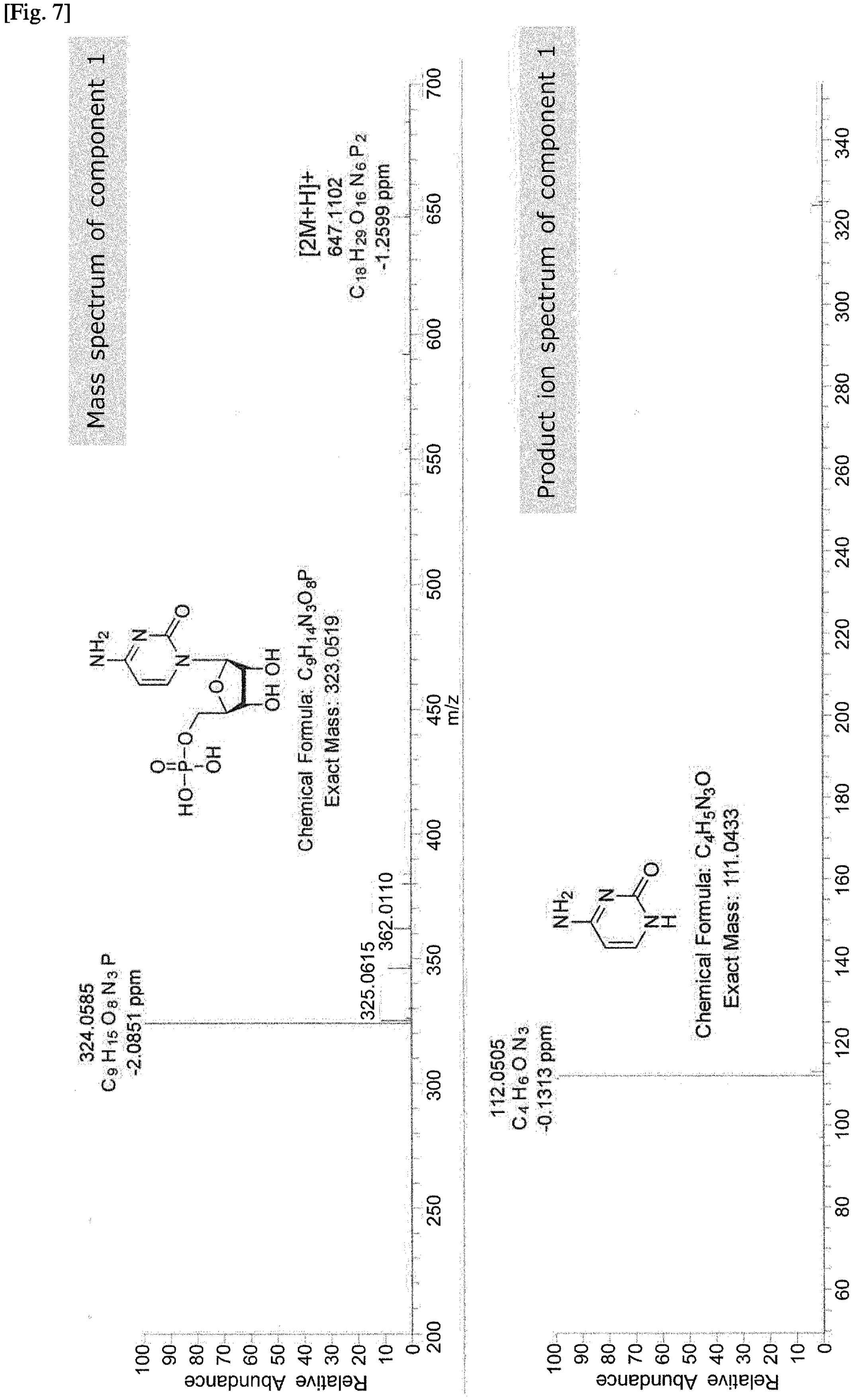
Mass spectrum of component 1
324.0585
$C_9H_{15}O_8N_3P$
-2.0851 ppm
325.0615
362.0110
Chemical Formula: $C_9H_{14}N_3O_8P$
Exact Mass: 323.0519
[2M+H]+
647.1102
$C_{18}H_{29}O_{16}N_6P_2$
-1.2599 ppm
Relative Abundance
m/z
Product ion spectrum of component 1
112.0505
$C_4H_6ON_3$
-0.1313 ppm
Chemical Formula: $C_4H_5N_3O$
Exact Mass: 111.0433
Relative Abundance
m/z

[Fig. 8]

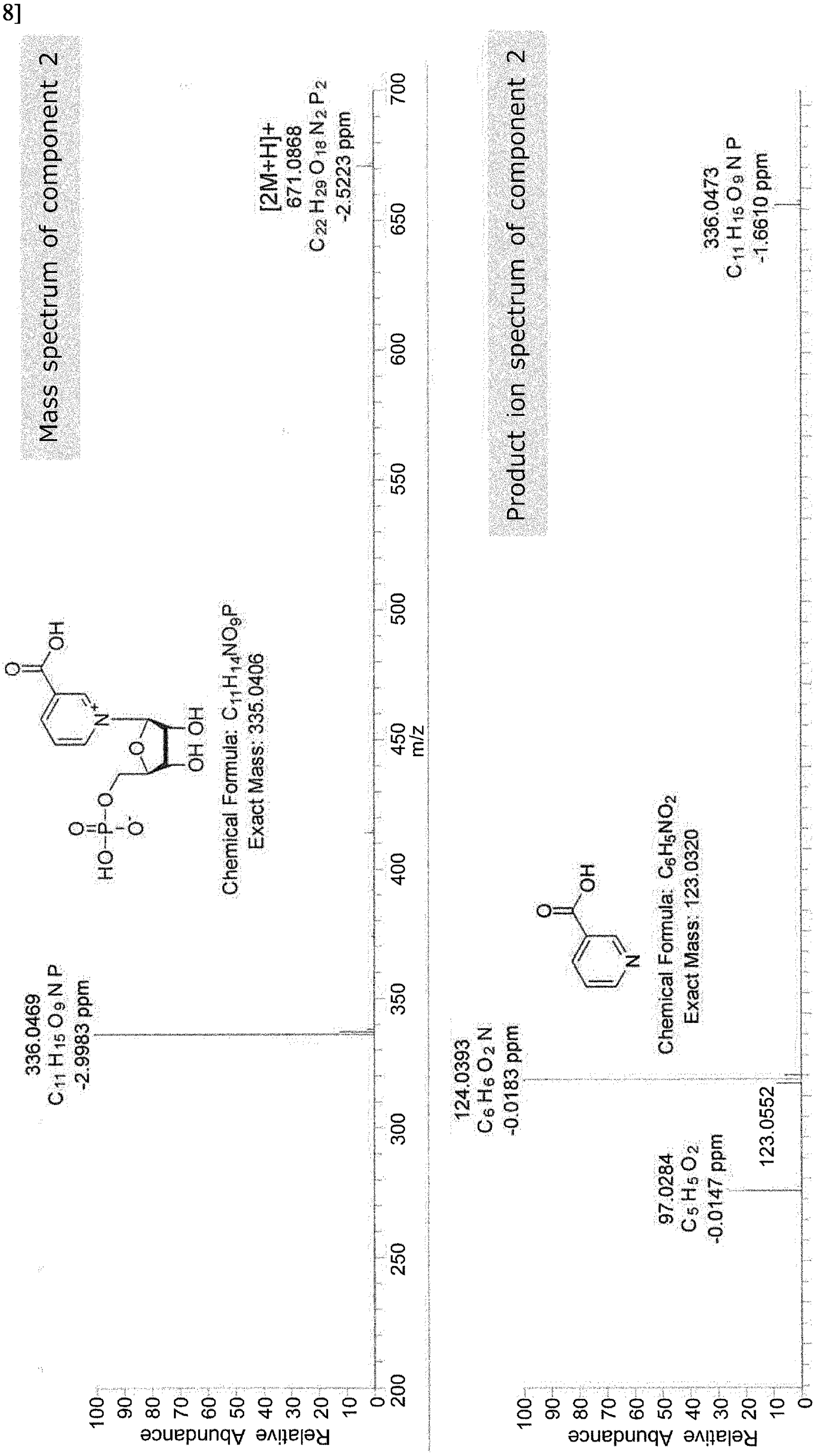
Mass spectrum of component 2
Relative Abundance
100 90 80 70 60 50 40 30 20 10 0
200 250 300 350 400 450 500 550 600 650 700
m/z
336.0469
C 11 H 15 O 9 N P
-2.9983 ppm
Chemical Formula: C11H14NO9P
Exact Mass: 335.0406
[2M+H]+
671.0868
C 22 H 29 O 18 N 2 P 2
-2.5223 ppm
Product ion spectrum of component 2
60 80 100 120 140 160 180 200 220 240 260 280 300 320 340 360
97.0284
C 5 H 5 O 2
-0.0147 ppm
123.0552
124.0393
C 6 H 6 O 2 N
-0.0183 ppm
Chemical Formula: C6H5NO2
Exact Mass: 123.0320
336.0473
C 11 H 15 O 9 N P
-1.6610 ppm

[Fig. 9]

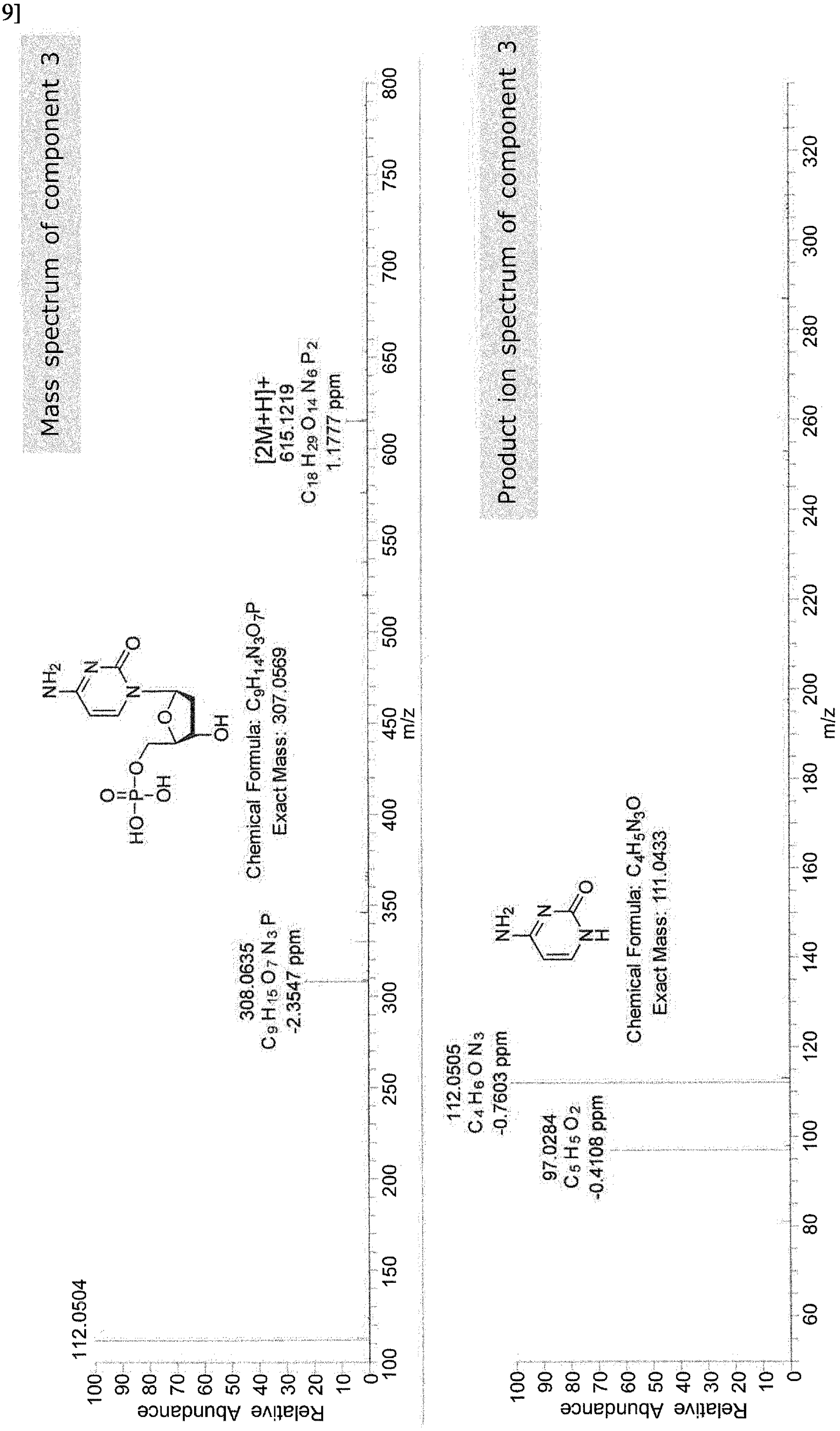
Mass spectrum of component 3
112.0504
308.0635
$C_9H_{15}O_7N_3P$
-2.3547 ppm
Chemical Formula: $C_9H_{14}N_3O_7P$
Exact Mass: 307.0569
[2M+H]+
615.1219
$C_{18}H_{29}O_{14}N_6P_2$
1.1777 ppm
Relative Abundance
m/z
Product ion spectrum of component 3
112.0505
$C_4H_6ON_3$
-0.7603 ppm
97.0284
$C_5H_5O_2$
-0.4108 ppm
Chemical Formula: $C_4H_5N_3O$
Exact Mass: 111.0433
Relative Abundance
m/z

[Fig. 10]

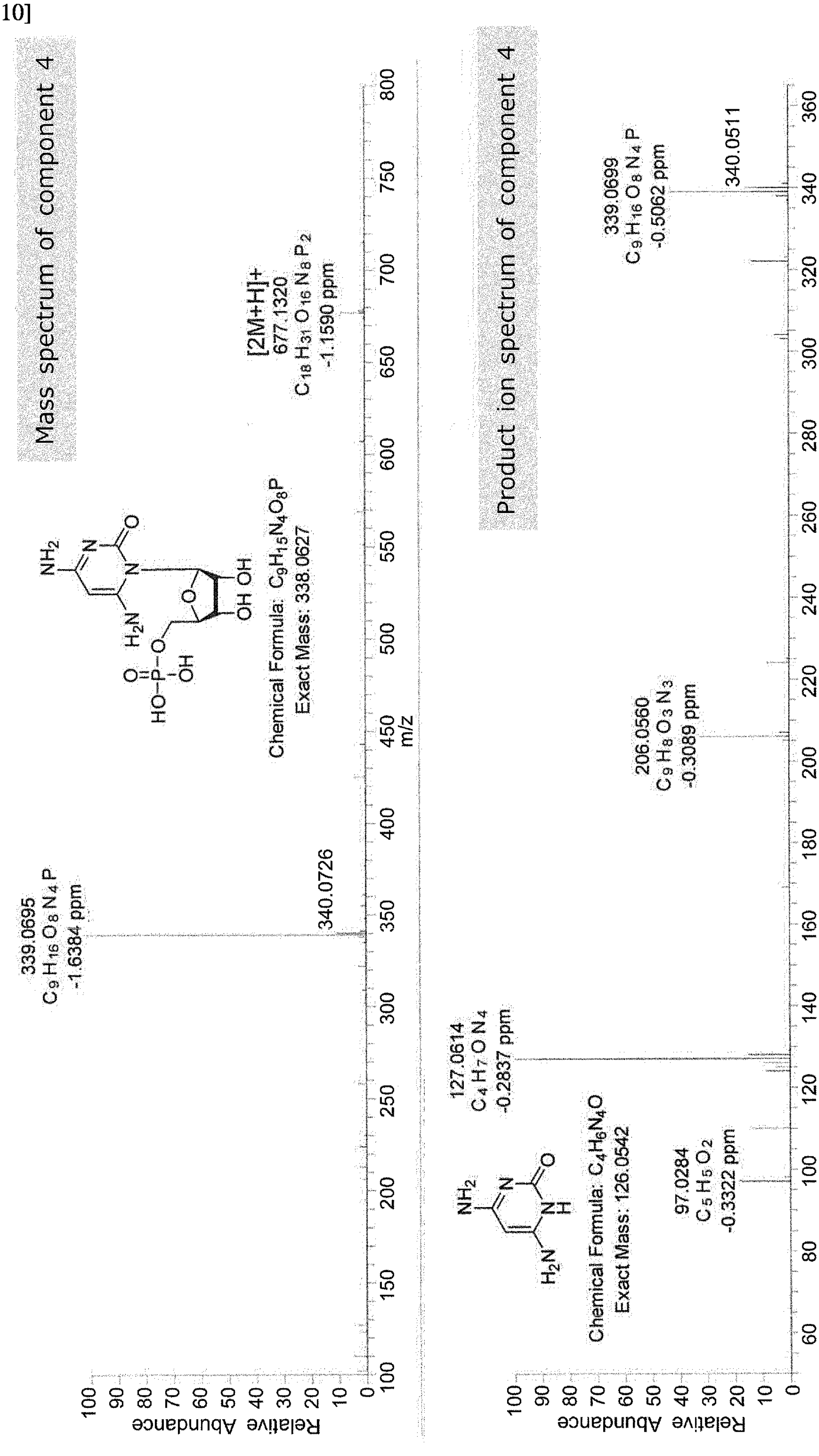
Mass spectrum of component 4
Relative Abundance
100
90
80
70
60
50
40
30
20
10
0
100
150
200
250
300
350
400
450
500
550
600
650
700
750
800
m/z
339.0695
C9 H16 O8 N4 P
-1.6384 ppm
340.0726
Chemical Formula: C9H15N4O8P
Exact Mass: 338.0627
[2M+H]+
677.1320
C18 H31 O16 N8 P2
-1.1590 ppm
Product ion spectrum of component 4
Chemical Formula: C4H6N4O
Exact Mass: 126.0542
97.0284
C5 H5 O2
-0.3322 ppm
127.0614
C4 H7 O N4
-0.2837 ppm
206.0560
C9 H8 O3 N3
-0.3089 ppm
339.0699
C9 H16 O8 N4 P
-0.5062 ppm
340.0511
60
80
100
120
140
160
180
200
220
240
260
280
300
320
340
360

[Fig. 11]

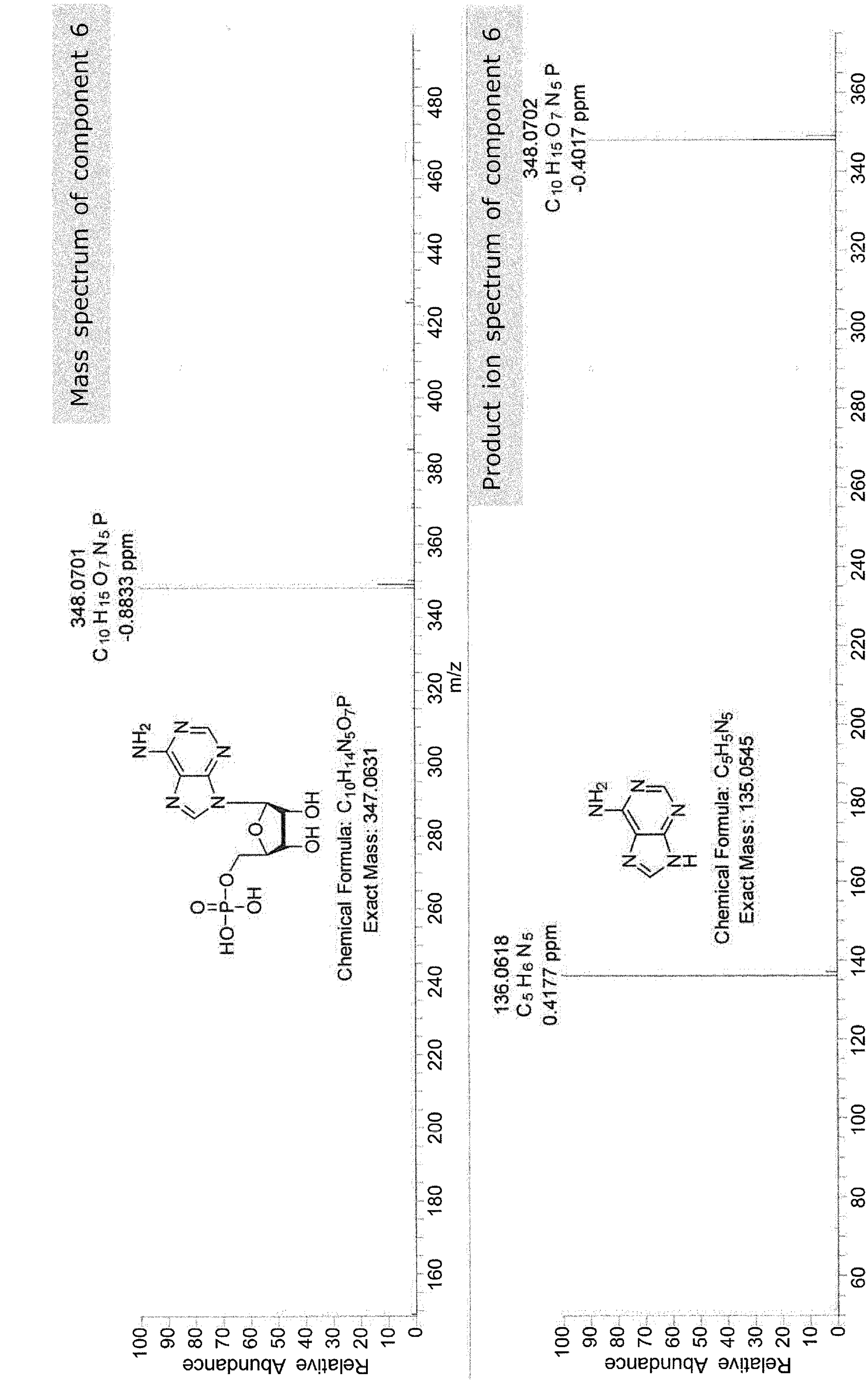
Mass spectrum of component 6
348.0701
$C_{10}H_{15}O_7N_5P$
-0.8833 ppm
Chemical Formula: $C_{10}H_{14}N_5O_7P$
Exact Mass: 347.0631
Relative Abundance
m/z
Product ion spectrum of component 6
348.0702
$C_{10}H_{15}O_7N_5P$
-0.4017 ppm
136.0618
$C_5H_6N_5$
0.4177 ppm
Chemical Formula: $C_5H_5N_5$
Exact Mass: 135.0545
Relative Abundance
m/z

[Fig. 12]

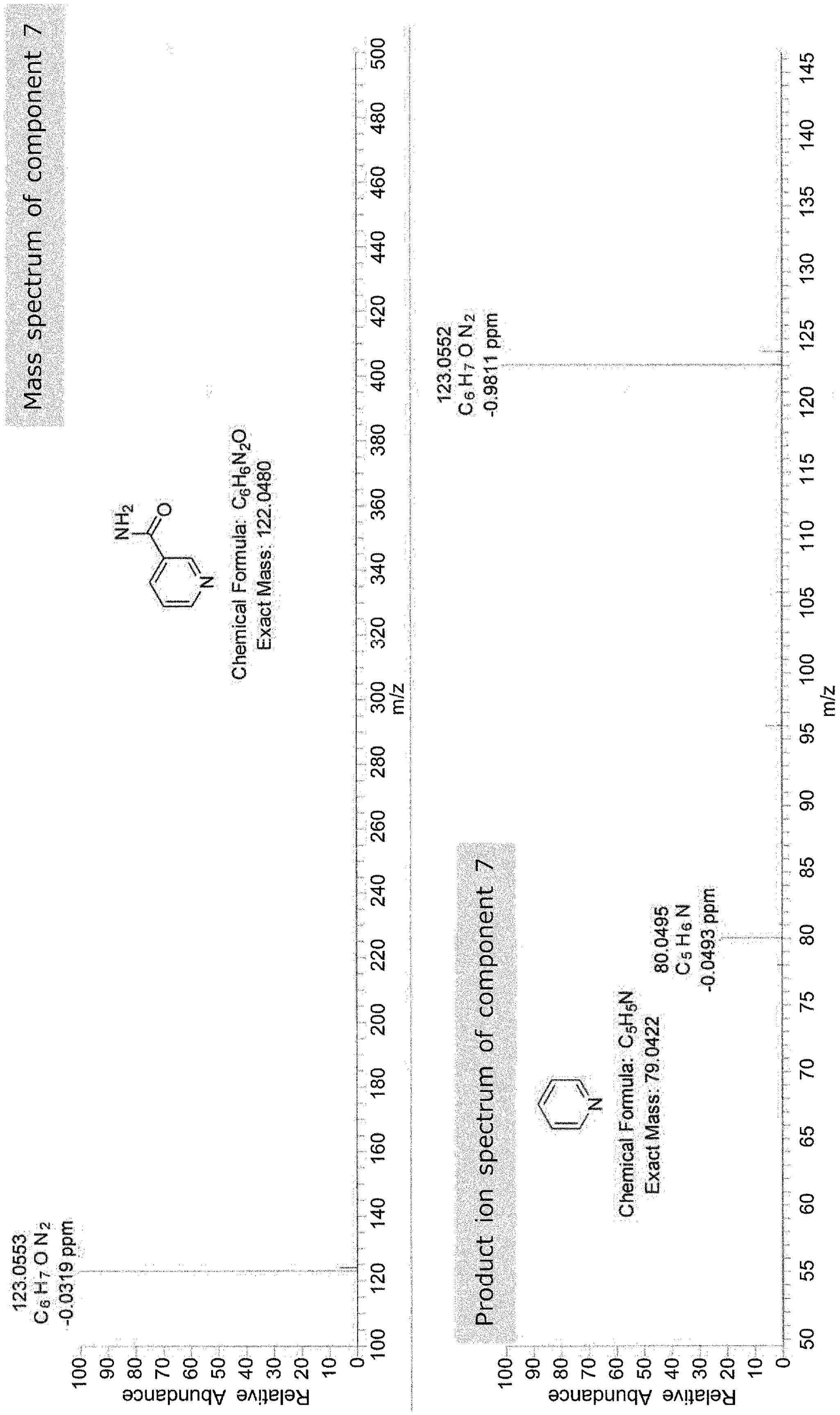
Mass spectrum of component 7
123.0553
C6 H7 O N2
-0.0319 ppm
Chemical Formula: C6H6N2O
Exact Mass: 122.0480
Relative Abundance
m/z
Product ion spectrum of component 7
123.0552
C6 H7 O N2
-0.9811 ppm
80.0495
C5 H6 N
-0.0493 ppm
Chemical Formula: C5H5N
Exact Mass: 79.0422
Relative Abundance
m/z

[Fig. 13]

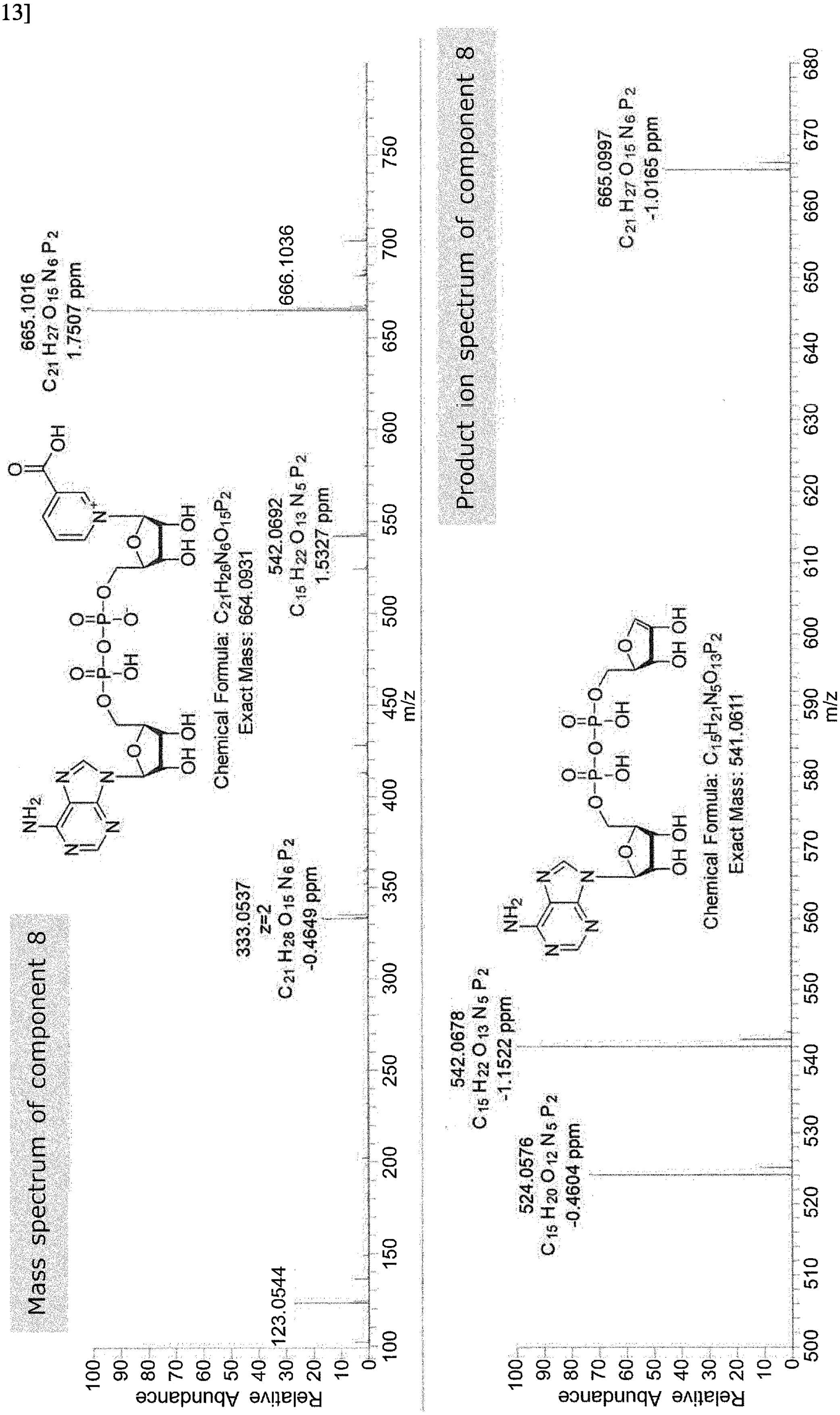
Mass spectrum of component 8
Relative Abundance
123.0544
333.0537
z=2
C 21 H 28 O 15 N 6 P 2
-0.4649 ppm
Chemical Formula: C21H26N6O15P2
Exact Mass: 664.0931
542.0692
C 15 H 22 O 13 N 5 P 2
1.5327 ppm
665.1016
C 21 H 27 O 15 N 6 P 2
1.7507 ppm
666.1036
m/z
Product ion spectrum of component 8
Relative Abundance
524.0576
C 15 H 20 O 12 N 5 P 2
-0.4604 ppm
542.0678
C 15 H 22 O 13 N 5 P 2
-1.1522 ppm
Chemical Formula: C15H21N5O13P2
Exact Mass: 541.0611
665.0997
C 21 H 27 O 15 N 6 P 2
-1.0165 ppm
m/z

[Fig. 14]

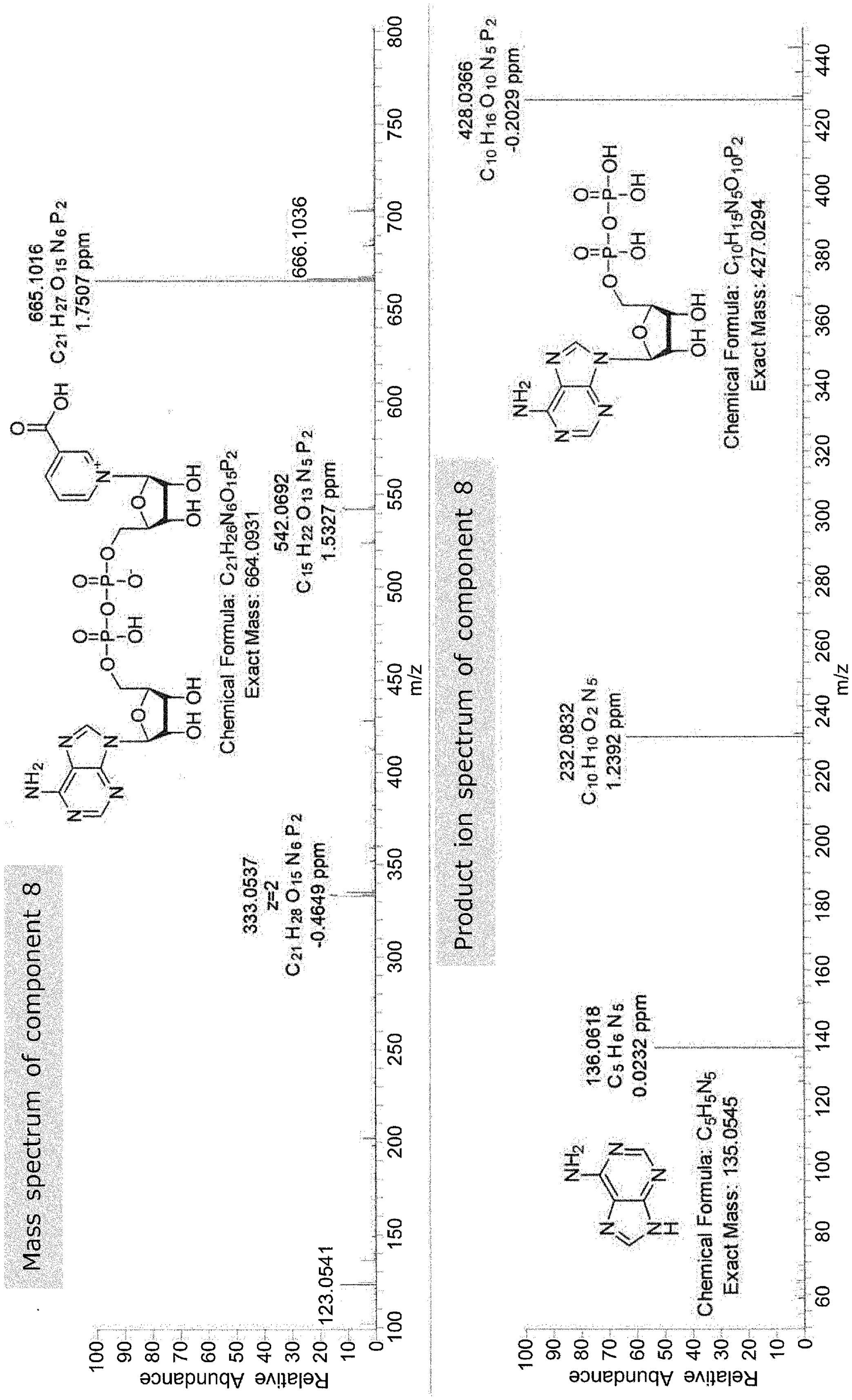
Mass spectrum of component 8
Relative Abundance
m/z
123.0541
333.0537
z=2
$C_{21}H_{28}O_{15}N_6P_2$
-0.4649 ppm
542.0692
$C_{15}H_{22}O_{13}N_5P_2$
1.5327 ppm
665.1016
$C_{21}H_{27}O_{15}N_6P_2$
1.7507 ppm
666.1036
Chemical Formula: $C_{21}H_{26}N_6O_{15}P_2$
Exact Mass: 664.0931
Product ion spectrum of component 8
136.0618
$C_5H_6N_5$
0.0232 ppm
Chemical Formula: $C_5H_5N_5$
Exact Mass: 135.0545
232.0832
$C_{10}H_{10}O_2N_5$
1.2392 ppm
428.0366
$C_{10}H_{16}O_{10}N_5P_2$
-0.2029 ppm
Chemical Formula: $C_{10}H_{15}N_5O_{10}P_2$
Exact Mass: 427.0294

## INTERNATIONAL SEARCH REPORT

International application No.
**PCT/JP2021/043333**

**A. CLASSIFICATION OF SUBJECT MATTER**

***C07H 19/048***(2006.01)i; ***A23L 33/13***(2016.01)i; ***A61K 31/706***(2006.01)i; ***A61P 3/00***(2006.01)i; ***A61P 5/00***(2006.01)i; ***A61P 9/10***(2006.01)i; ***A61P 43/00***(2006.01)i
FI: C07H19/048; A61K31/706; A61P43/00 107; A61P3/00; A61P5/00; A61P9/10 101; A23L33/13

According to International Patent Classification (IPC) or to both national classification and IPC

**B. FIELDS SEARCHED**

Minimum documentation searched (classification system followed by classification symbols)

C07H19/048; A23L33/13; A61K31/706; A61P3/00; A61P5/00; A61P9/10; A61P43/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2018/023205 A1 (BONTAC BIO-ENGINEERING (SHENZHEN) CO., LTD) 08 February 2018 (2018-02-08) claims, paragraph [0002], examples, table 1 | 1-3 |
| Y | | 4 |
| Y | JP 2019-525760 A (BONTAC BIO-ENGINEERING (SHENZHEN) CO., LTD.) 12 September 2019 (2019-09-12) claims, paragraph [0022], example 8 | 4 |
| Y | JP 2014-527955 A (ASTRAZENECA AB) 23 October 2014 (2014-10-23) paragraph [0161] | 4 |
| Y | JP 2009-538275 A (ORTHO-MCNEIL PHARMACEUTICAL, LLC.) 05 November 2009 (2009-11-05) paragraph [0160] | 4 |
| Y | JP 2009-515935 A (SANOFI-AVENTIS) 16 April 2009 (2009-04-16) paragraph [0098] | 4 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/043333**

| Patent document cited in search report | Publication date (day/month/year) | Patent family member(s) | Publication date (day/month/year) |
|---|---|---|---|
| WO 2018/023205 A1 | 08 February 2018 | CN 108026132 A | |
| JP 2019-525760 A | 12 September 2019 | US 2018/0230443 A1<br>claims, paragraph [0022], example 8<br>WO 2018/023206 A1<br>CN 108026517 A | |
| JP 2014-527955 A | 23 October 2014 | WO 2013/001294 A1<br>p. 34, lines 21-26<br>EP 2726464 A1<br>KR 10-2014-0036230 A<br>CN 103814015 A | |
| JP 2009-538275 A | 05 November 2009 | US 2007/0254957 A1<br>paragraph [0220]<br>WO 2007/124351 A1<br>EP 1847524 A1<br>KR 10-2009-0024678 A<br>CN 101484410 A | |
| JP 2009-515935 A | 16 April 2009 | US 2008/0275102 A1<br>paragraph [0283]<br>WO 2007/057571 A2<br>EP 1951225 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015069860 A
- WO 2017185549 A


### Non-patent literature cited in the description


- **YOSHINO, J. ; MILLS, K.F. ; YOON, M.J. ; IMAI, S.** *Cell Metab.,* 2011, vol. 14, 528-536
- **GOMES, A.P. et al.** *Cell,* 2013, vol. 155, 1624-1638
- **STEIN, L.R. ; IMAI, S.** *EMBO J.,* 2014, vol. 33, 1321-1340